# EUROPEAN PATENT SPECIFICATION

(11) **EP 0 705 270 B1**
(45) Date of publication and mention of the grant of the patent: **29.12.1997**
(21) Application number: 94923258.1
(22) Date of filing: 24.06.1994
(51) Int. Cl.: C07H 21/00, C12Q 1/68

(54) **CHEMICALLY MODIFIED OLIGONUCLEOTIDE FOR SITE-DIRECTED MUTAGENESIS**
CHEMISCHE-MODIFIZIERTE OLIGONUKLEOTIDE FÜR SITE-SPEZIFISCHES MUTAGENESIS
OLIGONUCLEOTIDE MODIFIE CHIMIQUEMENT EN VUE D'UNE MUTAGENESE DIRIGEE VERS LE SITE

(30) Priority: 25.06.1993 US 83088
(43) Date of publication of application: 10.04.1996
(73) Proprietor: YALE UNIVERSITY, New Haven, CT 06511 (US)
(72) Inventor: GLAZER, Peter, M., Guilford, CT 06437 (US); HAVRE, Pamela, A. 433 Whitney Avenue, New Haven, CT 06511 (US)
(74) Representative: Bassett, Richard Simon
(86) International application number: PCT/US94/07234
(87) International publication number: WO 95/01364

(56) References cited:
- EP-A- 0 266 099
- EP-A- 0 375 408
- PROCEEDINGS OF THE NATIONAL ACADEMY OF SCIENCES OF USA., vol.88, no.13, 1 July 1991, WASHINGTON US pages 5602 - 5606 M.TAKASUGI ET AL. 'Sequence-specific Photo-induced Cross-linking of the two Strands of Double-helical DNA by a Psoralen Covalently Linked to a Triple Helix Froming Oligonucleotide.' cited in the application
- PROCEEDINGS OF THE NATIONAL ACADEMY OF SCIENCES OF USA., vol.90, no.8, 15 April 1993, WASHINGTON US pages 3501 - 3505 M.GRIGORIEV ET AL. 'Inhibition of Gene Expression by Triple Helix-directed DNA Cross-linking at Specific Sites.'
- NUCLEIC ACIDS RESEARCH., vol.20, no.16, 25 August 1992, ARLINGTON, VIRGINIA US pages 4275 - 4281 C.GIOVANNANGELI ET AL. 'Oligodeoxynucleotide-directed Photo-induced Cross-linking of HIV Proviral DNA via Triple-helix Formation.'
- PROCEEDINGS OF THE NATIONAL ACADEMY OF SCIENCES OF USA., vol.90, no.16, 15 August 1993, WASHINGTON US pages 7879 - 7883 P.A.HAVRE ET AL. 'Targeted Mutagenesis of DNA using Triple Helix Forming Oligonucleotides Linked to Psoralen.'
- NUCLEIC ACIDS RESEARCH., vol.22, no.14, 25 July 1994, ARLINGTON, VIRGINIA US pages 2845 - 2852 F.P.GASPARRO ET AL. 'Site-Specific Targeting of Psoralen Photoadducts with a Triple Helix-Forming Oligonucleotide: Characterization of Psoralen Monoadduct and Crosslink Formation.'

## Description

### Background of the Invention

This relates to the fields of genetics, and more particularly relates to site-directed mutagenesis of a gene of interest.

### Gene Therapy

Gene therapy is the introduction into a cell of an entire replacement copy of a defective gene to treat human, animal and plant genetic disorders. The introduced gene, via genetic recombination, replaces the endogenous gene. This approach requires complex delivery systems to introduce the replacement gene into the cell, such as genetically engineered viruses, or viral vectors.

Gene therapy is being used on an experimental basis to treat well known genetic disorders of humans such as retinoblastoma, cystic fibrosis, and sickle cell anemia. However, *in vivo* efficiency is low due to the limited number of recombination events actually resulting in replacement of the defective gene.

### Triple-stranded DNA

Since the initial observation of triple-stranded DNA many years ago by Felsenfeld et al., *J. Am. Chem. Soc.* 79:2023 (1957), oligonucleotide-directed triple helix formation has emerged as a valuable tool in molecular biology. Current knowledge suggests that oligonucleotides can bind as third strands of DNA in a sequence specific manner in the major groove in polypurine/polypyrimidine stretches in duplex DNA. In one motif, a polypyrimidine oligonucleotide binds in a direction parallel to the purine strand in the duplex, as described by Moser and Dervan, *Science* 238:645 (1987), Praseuth et al., *Proc. Natl. Acad. Sci. USA* 85:1349 (1988), and Mergny et al., *Biochemistry* 30:9791 (1991). In the alternate purine motif, a polypurine strand binds anti-parallel to the purine strand, as described by Beal and Dervan, *Science* 251:1360 (1991). The specificity of triplex formation arises from base triplets (AAT and GGC in the purine motif) formed by hydrogen bonding; mismatches destabilize the triple helix, as described by Mergny et al., *Biochemistry* 30:9791 (1991) and Beal and Dervan, *Nuc. Acids Res.* 11:2773 (1992).

Triplex forming oligonucleotides have been found useful for several molecular biology techniques. For example, triplex forming oligonucleotides designed to bind to sites in gene promoters have been used to block DNA binding proteins and to block transcription both *in vitro* and *in vivo.* (Maher et al., *Science* 245:725 (1989), Orson et al., *Nucleic Acids Res.* 19:3435 (1991), Postal et al., *Proc. Natl. Acad. Sci. USA* 88:8227 (1991), Cooney et al., *Science* 241:456 (1988), Young et al., *Proc. Natl. Acad. Sci. USA* 88:10023 (1991), Maher et al., *Biochemistry* 31:70 (1992), Duval-Valentin et al., Proc. Natl. Acad. Sci. USA 89:504 (1992), Blume et al., *Nucleic Acids Res.* 20:1777 (1992), Durland et al., *Biochemistry* 30:9246 (1991), Grigoriev et al., *J. of Biological Chem.* 267:3389 (1992), and Takasugi et al., *Proc. Natl. Acad. Sci. USA* 88:5602 (1991)). Site specific cleavage of DNA has been achieved by using triplex forming oligonucleotides linked to reactive moieties such as EDTA-Fe(II) or by using triplex forming oligonucleotides in conjunction with DNA modifying enzymes (Perrouault et al., *Nature* 344:358 (1990), Francois et al., *Proc. Natl. Acad.* Sci. USA 86:9702 (1989), Lin et al., *Biochemistry* 28:1054 (1989), Pei et al. *Proc. Natl. Acad. Sci. USA* 87:9858 (1990), Strobel et al., *Science* 254:1639 (1991), and Posvic and Dervan, *J. Am. Chem Soc.* 112:9428 (1992)). Sequence specific DNA purification using triplex affinity capture has also been demonstrated. (Ito et al., *Proc. Natl. Acad. Sci. USA* 89:495 (1992)). Triplex forming oligonucleotides linked to intercalating agents such as acridine, or to cross-linking agents, such as p-azidophenacyl and psoralen, have been utilized, but only to enhance the stability of triplex binding. (Praseuth et al., *Proc. Natl. Acad. Sci. USA* 85:1349 (1988), Grigoriev et al., J. *of Biological Chem.* 267:3389 (1992), Takasugi et al., *Proc. Natl. Acad. Sci. USA* 88:5602 (1991).

EP 0 266 099 describes oligonucleoside alkyl or arylphosphonate derivatives capable of crosslinking with or cleaving nucleic acids.

EP 0 375 408 describes methods for making synthetic oligonucleotides which bind specifically to target sites on duplex DNA molecules.

Grigorier *et al* (1993) *Proc. Natl. Acad. Sci. USA* **90**, 3501-3505 describes the inhibition of gene expression by triple helix-directed DNA cross-linking at specific sites.

Giovannangeli *et al* (1992) *Nucl. Acids Res.* **20**, 4275-4281 describes oligodeoxynucleotide-directed photo-induced cross-linking of HIV proviral DNA *via* triple-helix formation.

A method for site-directed mutagenesis of a target DNA molecule would be a useful in achieving successful gene or anti-viral therapy. Such a method would also be a useful research tool for genetic engineering or for studying genetic mechanisms such as DNA repair.

Therefore, it is an object of the present invention to provide a method for *in vivo* and *in vitro* site-directed mutagenesis of a target DNA molecule.

It is a further object of the present invention to provide a method for mutagenesis of a target DNA molecule that is highly specific and efficient.

It is a further object of the present invention to provide a method for treating genetic disorders by gene therapy without the need for a viral vector.

It is a further object of the present invention to provide a method for treating cancer.

It is a further object of the present invention to provide a mutagenic oligonucleotide for use in therapy and research.

A first aspect of the invention provides a composition comprising a mutagenic oligonucleotide for site-directed mutagenesis of a double-stranded nucleic acid molecule comprising a mutagen incorporated into a single-stranded oligonucleotide between 7 and 40 nucleotides in length having a sequence that forms a triple-stranded nucleic acid molecule with a target region of the double-stranded nucleic acid molecule, and a pharmaceutically acceptable carrier for administration to cells.

A second aspect of the invention provides an *ex vivo* method for site-directed mutagenesis of a nucleic acid molecule *in situ* in a cell comprising the steps of: a) selecting a target region of the nucleic acid molecule; b) introducing a mutagenic oligonucleotide into the cell; c) hybridizing a mutagenic oligonucleotide to a target region of a double-stranded nucleic acid molecule, wherein the mutagenic oligonucleotide comprises a mutagen incorporated into a single-stranded oligonucleotide between 7 and 40 nucleotides in length that forms a triple-stranded nucleic acid molecule with the target region; and d) mutating the double-stranded nucleic acid molecule.

A third aspect of the invention provides a method of producing a composition of the first aspect of the invention the method comprising the steps of: a) synthesizing an oligonucleotide substantially complementary based on the third strand binding code to a target region of a double-stranded nucleic acid molecule; and b) incorporating a mutagen in the oligonucleotide, and c) admixing the mutagenic oligonucleotide with a pharmaceutically acceptable carrier for administration to cells.

A fourth aspect of the invention provides a mutagenic oligonucleotide for site-directed mutagenesis of a double-stranded nucleic acid molecule comprising a mutagen incorporated into a single-stranded oligonucleotide between 7 and 40 nucleotides in length having a sequence that forms a triple-stranded nucleic acid molecule with a target region of the double-stranded nucleic acid molecule for use in medicine.

A fifth aspect of the invention provides the use of a mutagenic oligonucleotide comprising a mutagen incorporated into a single-stranded oligonucleotide between 7 and 40 nucleotides in length in the manufacture of a medicament for treating a cell or a patient which cell or which patient will benefit from site-directed mutagenesis of a double-stranded nucleic acid molecule, wherein the target region of said nucleic acid molecule is selected prior to treatment and the said oligonucleotide has a sequence that forms a triple-stranded nucleic acid molecule with a target region of the double-stranded nucleic acid molecule.

A sixth aspect provides use of an oligonucleotide as defined in the first aspect of the invention for *ex vivo* site-directed mutagenesis of a nucleic acid molecule *in situ* in a cell.

A mutagenic, triplex-forming oligonucleotide and methods for use thereof are described herein. An oligonucleotide capable of forming a triple strand with a specific DNA segment of a target gene DNA is chemically modified to incorporate a mutagen. The modified oligonucleotide hybridizes to a chosen site in the target gene, forming a triplex region, thereby bringing the attached mutagen into proximity with the target gene and causing a mutation at a specific site in the gene. The mutation activates, inactivates, or alters the activity and function of the target gene.

If the target gene contains a mutation that is the cause of a genetic disorder, then the mutagenic oligonucleotide is useful for mutagenic repair that may restore the DNA sequence of the target gene to normal. If the target gene is a viral gene needed for viral survival or reproduction or an oncogene causing unregulated proliferation, such as in a cancer cell, then the mutagenic oligonucleotide is useful for causing a mutation that inactivates the gene to incapacitate or prevent reproduction of the virus or to terminate or reduce the uncontrolled proliferation of the cancer cell. The mutagenic oligonucleotide is also a useful anti-cancer agent for activating a repressor gene that has lost its ability to repress proliferation.

The mutagenic triplex-forming oligonucleotide is also particularly useful as a molecular biology research tool to cause site-directed or targeted mutagenesis. Site-directed mutagenesis is useful for targeting a normal gene and for the study of mechanisms such as DNA repair. Targeted mutagenesis of a specific gene in an animal oocyte, such as a mouse oocyte, provides a useful and powerful tool for genetic engineering for research and therapy and for generation of new strains of "transmutated" animals and plants for research and agriculture.

### Brief Description of the Drawings

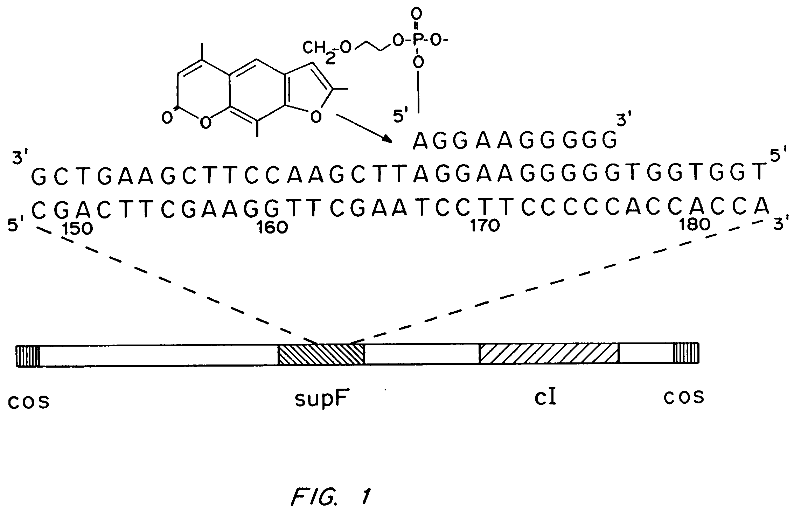

Figure 1 is a schematic representation showing a psoralen-linked triplex-forming oligonucleotide for targeted mutagenesis of the lambda genome. A map of the lambda supF genome is shown, including the target gene for site-directed mutagenesis, the supF suppressor tRNA gene. Above the partial sequence of the supF gene (positions 149 to 183), the site of triplex formation at positions 167-176 is indicated by the placement of the triplex-forming oligonucleotide, pso-AG10 (4'hydroxymethyl-4,5',8-trimethylpsoralen-⁵'AGGAAGGGGG³'). The arrow indicates that the psoralen moiety is targeted to the A:T base pair at position 167. In addition to the supF gene, the lambda vector carries the cI lambda repressor gene which is used to assess non-targeted mutagenesis.

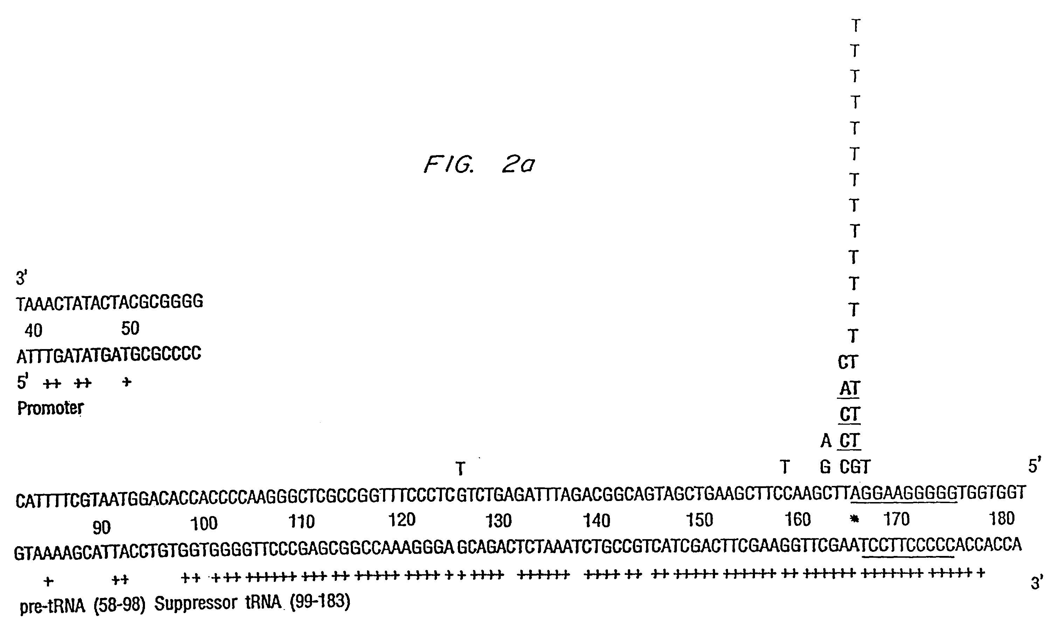

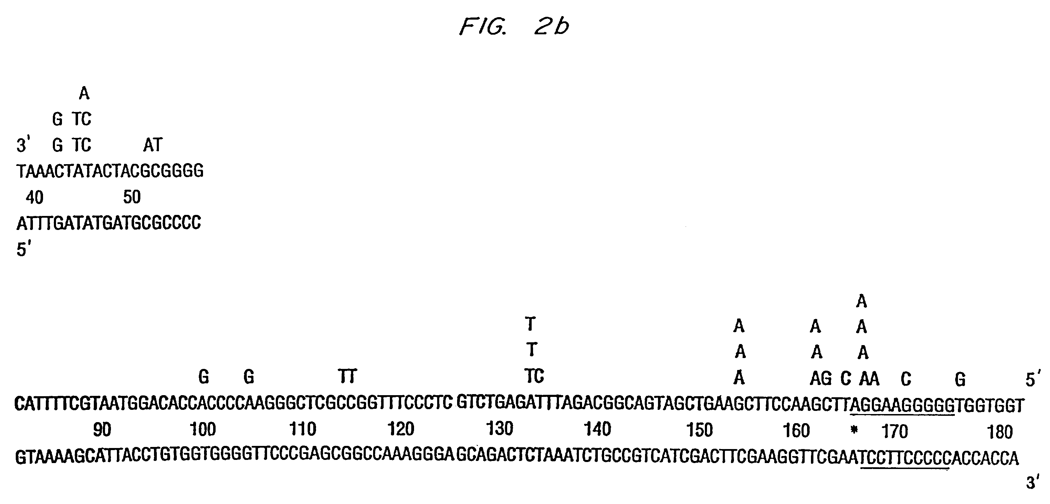

Figures 2a and 2b show a sequence analysis of targeted mutagenesis in the supF gene by the psoralen-linked triplex-forming oligonucleotide (pso-AG10). In Figure 2a, mutations produced by pso-AG10 and UVA are indicated above each base pair, with the listed base representing the change from the sequence in the top strand. The + signs below the sequence are sites at which mutations are known to produce a detectable phenotype change, demonstrating that the use of supF in this assay does not bias detection at any particular site. The asterisk indicates the targeted base pair at position 167. DNA sequence data was obtained by automated methods after polymerase chain reaction amplification of the supF genes from lambda phage plaques in accordance with the method of Connell et al., *Biotechniques* 5:342 (1987). Figure 2b is a compilation of mutations induced in supF by 8-methoxypsoralen and UVA in mouse L-cells using the lambda supF vector of Figure 1 or generated using a plasmid shuttle vector in monkey Vero cells in accordance with the method of Bredberg and Nachmansson, *Carcinogenesis* 8:1923 (1987), to compare the mutations produced in supF by free psoralen with those produced by the triplex forming oligonucleotide AG10.

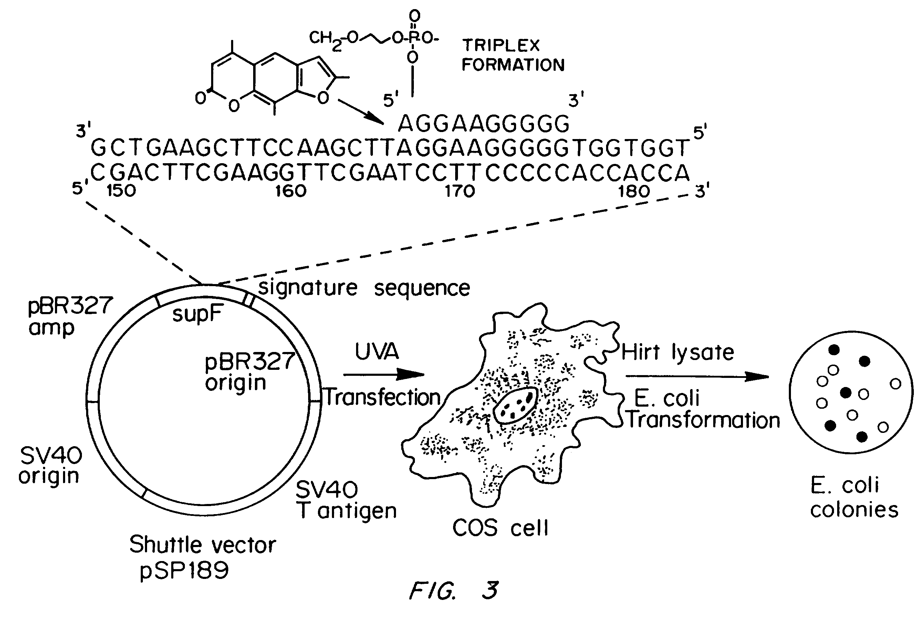

Figure 3 is a schematic representation of the strategy for targeted mutagenesis of SV40 DNA. The 10 base triplex-forming oligonucleotide, psoralen-AG10 (4'hydroxymethyl-4,5',8-trimethyl-5'AGGAAGGGGG3'), is shown directly above its targeted sequence in the supF gene (base pairs 167-176), contained within the SV40 vector, pSP189. Psoralen-AG10 is incubated with the SV40 vector DNA to allow site-specific triplex formation. Photoactivation of the psoralen by irradiation with long wave ultraviolet light (320-400 nm) is designed to generate an adduct at the targeted base pair (167), as indicated by the arrow. The oligo-plasmid complex is then transfected into monkey COS-7 cells and allowed to replicate for 48 hours. Following purification of the vector DNA by the Hirt lysate procedure (Hirt et al., *J. Mol. Biol.* 26:365-369 (1967)), the DNA is used to transform E. coli SY204 *lacZ*125 (Am). Transformants are selected on ampicillin plates containing X-gal and isopropylthio-β-D-galactoside (IPTG) for detection and isolation of mutants (white colonies) in which the supF gene has been inactivated by mutation.

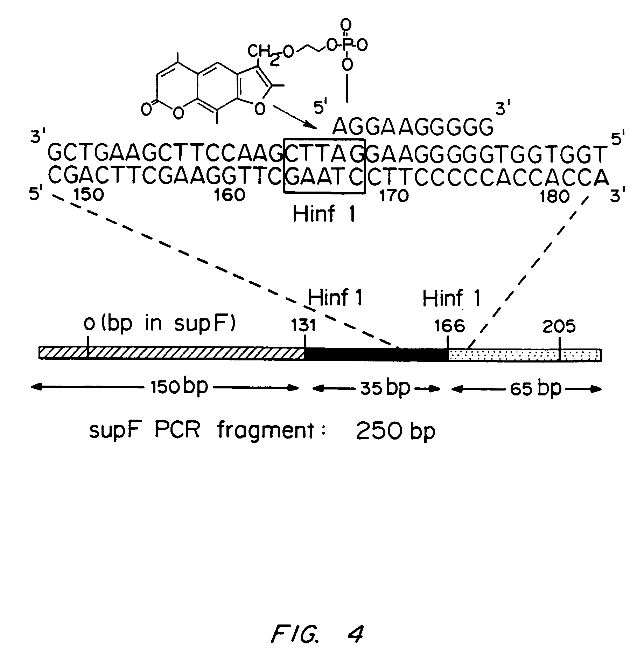

Figure 4 is a schematic representation showing the basis of a restriction enzyme protection assay, using *Hin*f I digestion, to detect site-specific triplex formation within the supF gene. The formation of triplex DNA by psoralen-AG10 at its targeted site (bp 167-176 of the supF gene) overlaps with the *Hin*f I restriction site at bp 164-168 (indicated in the diagram by the box around the appropriate base pairs). Digestion of the unprotected 250 bp supF PCR fragment with *Hinf* I is expected to yield three fragments of sizes 150, 65, and 35 bp. In contrast, with the *Hin*f I site at bp 164-168 blocked by triplex formation at bp 167-176, fragments of sizes 150 and 100 bp are predicted.

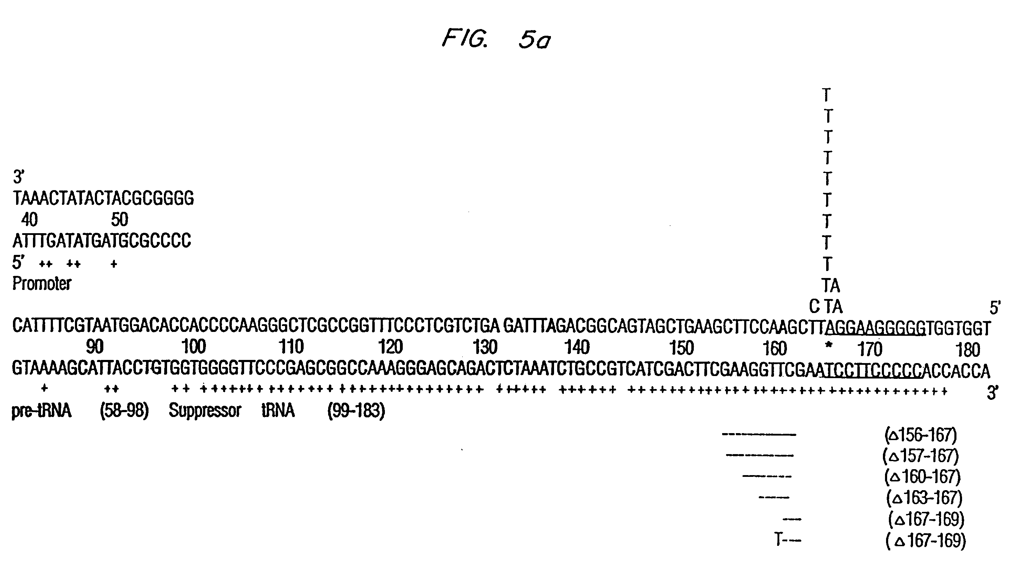

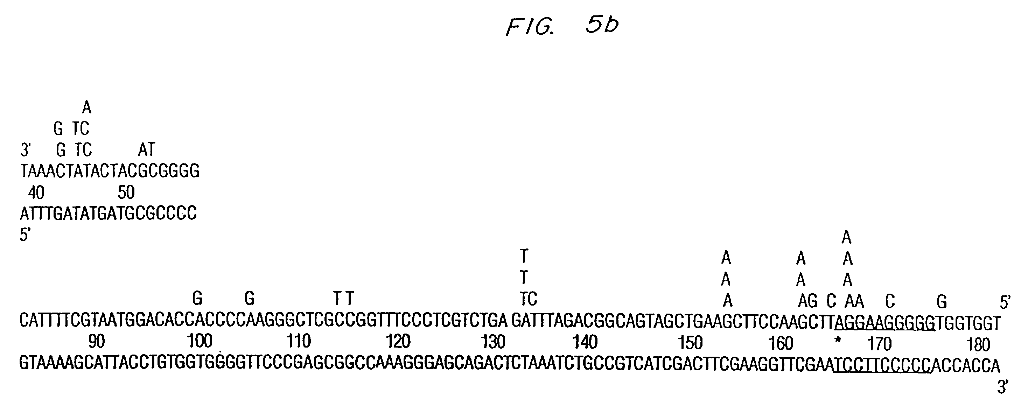

Figures 5A and 5B are a sequence analysis of targeted mutagenesis in the supF gene within the pSP189 SV40 vector by the psoralen-linked triplex-forming oligonucleotide, psoralen-AG10. In Figure 5A, point mutations produced by psoralen-AG10 and UVA are indicated above each base pair, with the listed base representing the change from the sequence in the top strand. Deletion mutations are presented below the supF sequence, indicated by dashed lines. For the one deletion that was accompanied by an apparent base change, the indicated base represents a mutation from the sequence of the top strand. The + signs below the sequence are sites at which mutations are known to produce a detectable phenotype change demonstrating that the use of supF in this assay does not bias detection at any particular site. The asterisk indicates the targeted base pair at position 167. Figure 5B is a compilation of mutations induced in supF by 8-methoxypsoralen and UVA in mouse L-cells using a lambda phage shuttle vector or generated in monkey Vero cells using an SV40 shuttle vector (pZ189) almost identical to the one used in this study to show for comparison the mutations that can be produced in supF by free psoralen.

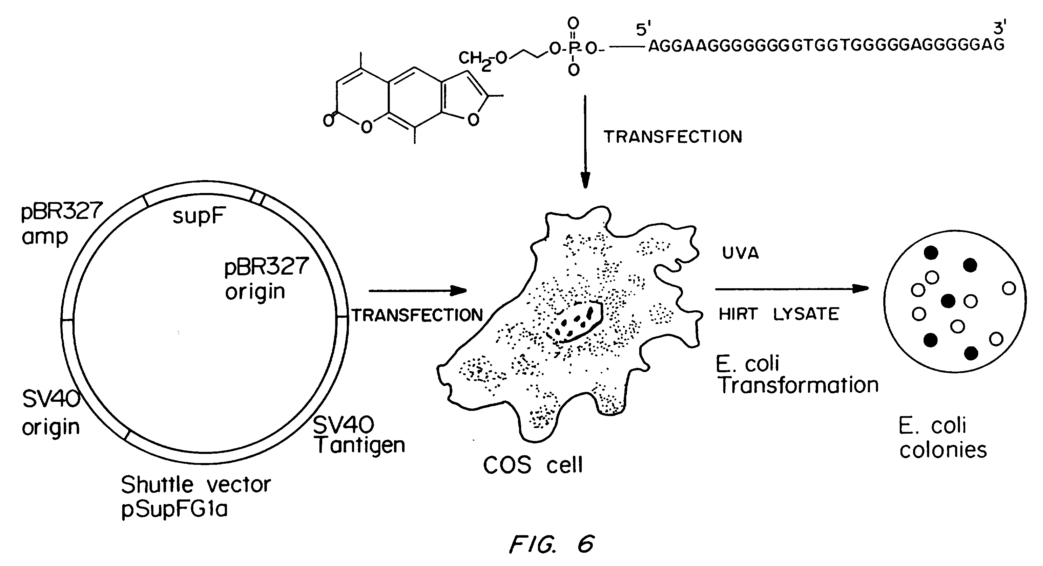

Figure 6 is a schematic representation of the strategy for targeted mutagenesis of SV40 DNA in monkey COS cells. Psoralen was incorporated by synthesis into a triplex-forming oligonucleotide as psoralen phosphoramidite. The SV40 shuttle vector, pSupFGl was a derivative of pSP189 and carried triplex-binding sites, which were engineered in to the *supF* gene. The plasmid is transfected into monkey COS-7 cells. Subsequently, the oligonucleotide is added to the cells, which are allowed to replicate for 48 hours. Photoactivation of the psoralen by irradiation with long wave ultraviolet light (320-400 nm) is designed to generate mutations. Following purification of the vector DNA by the Hirt lysate procedure (Hirt et al., *J. Mol. Biol.* 26:365-369 (1967)), the DNA is used to transform E. coli SY204 *lacZ*125 (Am). Transformants are selected on ampicillin plates containing X-gal and IPTG for detection and isolation of mutants (white colonies) in which the supF gene has been inactivated by mutation.

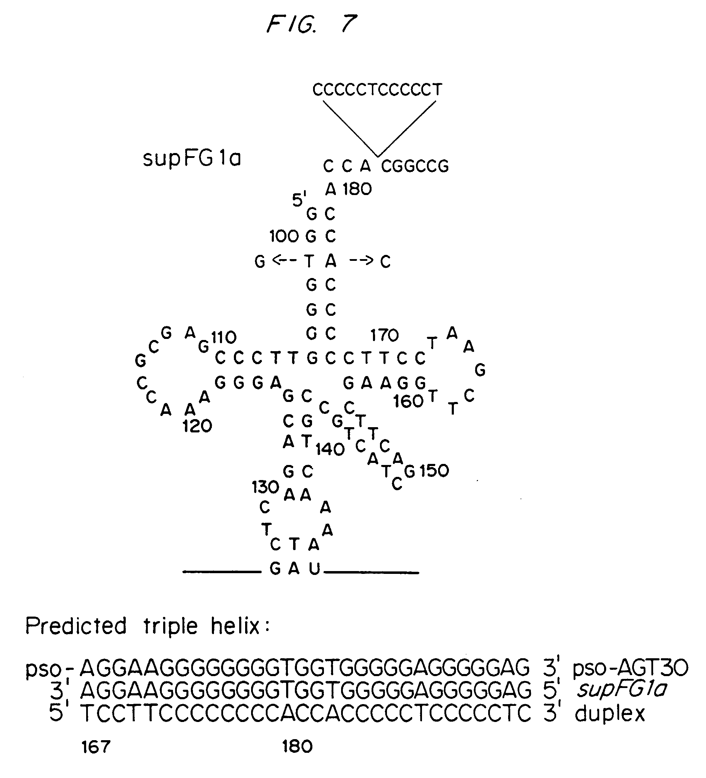

Figure 7 is the nucleotide sequence and two-dimensional structure of a modified *supF* gene, designated *supFG1a.* An A:T to C:G transversion was incorporated into the sequence, along with a compensatory T:A to G:C change at bp 101 to maintain base pairing in the amino acid acceptor stem of the mature tRNA thereby eliminating an interruption at bp 167 in the polypurine/polypyrimidine run. In addition, a 13 bp polypurine/polypyrimidine sequence was inserted between bp 183 and 184 to extend the length of the polypurine/polypyrimidine run in the gene to 30 bp. This construct contains a 30 bp polypurine site with two interruptions.

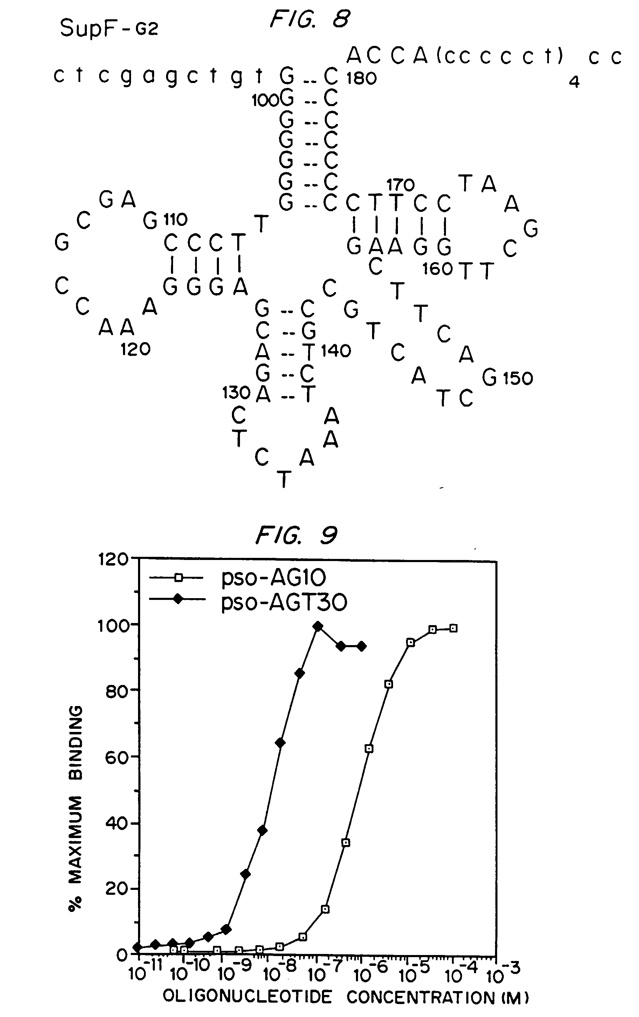

Figure 8 is the nucleotide sequence and two-dimensional structure of a modified *supF* gene, designated *supFG2.* This sequence contains a 43 bp polypurine site and two interruptions.

Figure 9 is a graph of percent maximum binding versus oligonucleotide concentration (M) of the mutagenic oligonucleotides pso-AG10 (open squares) and pso-AGT30 (diamonds).

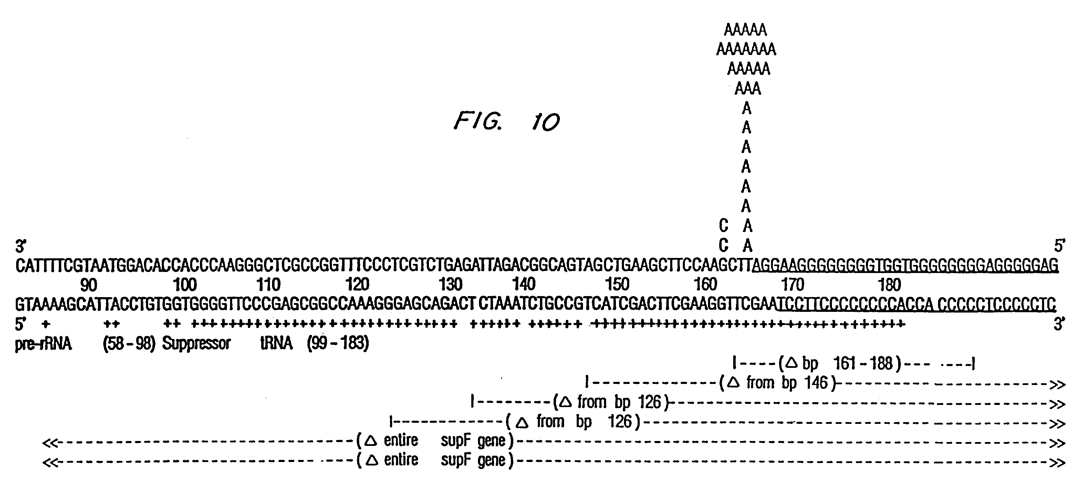

Figure 10 is a sequence analysis of sequences of mutations targeted to the supF gene within an SV40 vector (pSupFGla) by treatment of COS cells with pso-AGT30 and UVA.

### Detailed Description of the Invention

A mutagenic triplex-forming oligonucleotide and methods of use in gene therapy, anti-viral therapeutics, scientific research, and genetic engineering of cells, animals and plants are provided. The mutagenic oligonucleotide binds with specificity to a chosen site in a target DNA molecule, forming a triplex region, thereby bringing the attached mutagen into proximity with the target site and causing a mutation therein. Preferably, the mutation activates, inactivates or alters the activity and function of a gene containing the target site.

### Oligonucleotide

The oligonucleotide is a synthetic or isolated oligonucleotide capable of binding or hybridizing with specificity to a predetermined region of a double-stranded DNA molecule to form a triple-stranded structure. Preferably, the predetermined region of the double-stranded molecule contains or is adjacent to the defective or essential portion of a target gene, such as the site of a mutation causing a genetic defect, a site causing oncogene activation, or a site causing the inhibition or inactivation of an oncogene suppressor. Most preferably, the gene is a human gene.

Preferably, the oligonucleotide is a single-stranded DNA molecule between 7 and 40 nucleotides in length, most preferably 10 to 20 nucleotides in length for *in vitro* mutagenesis and 20 to 30 nucleotides in length for *in vivo* mutagenesis. The base composition is preferably homopurine or homopyrimidine. Alternatively, the base composition is polypurine or polypyrimidine. However, other compositions are also useful. The preferred conditions under which a triple-stranded structure will form and the desired nucleotide composition of the third strand are well known to those skilled in the art. (See for example, Moser and Dervan, *Science* 238:645 (1987); Praseuth et al., *Proc. Natl. Acad. Sci. USA* 85:1349 (1988); Mergny et al., *Biochemistry* 30:9791 (1991); Beal and Dervan, *Science* 251:1360 (1991); Mergny et al., *Biochemistry* 30:9791 (1991) and Beal and Dervan, *Nuc. Acids Res.* 11:2773 (1992).)

Preferably, the mutagenic oligonucleotide hybridizes to the target nucleic acid molecule under conditions of high stringency and specificity. Most preferably, the oligonucleotide binds in a sequence-specific manner in the major groove of duplex DNA. Reaction conditions for *in vitro* triple helix formation of an oligonucleotide probe or primer to a nucleic acid sequence vary from oligonucleotide to oligonucleotide, depending on factors such as oligonucleotide length, the number of G:C and A:T base pairs, and the composition of the buffer utilized in the hybridization reaction. A mutagenic oligonucleotide substantially complementary, based on the third strand binding code, to the target region of the double-stranded nucleic acid molecule is preferred.

A useful measure of triple helix formation is the equilibrium dissociation constant, K_{d}, of the triplex, which can be estimated as the concentration of mutagenic oligonucleotide at which triplex formation is half-maximal. Preferably, the oligonucleotide has a binding affinity for the target sequence in the range of physiologic interactions. The preferred mutagenic oligonucleotide has a K_{d} less than or equal to approximately 8 X 10⁻⁷ M. Most preferably, the K_{d} is less than or equal to 8 X 10⁻⁹ M in order to achieve significant intracellular interactions.

### Mutagen

The oligonucleotide is chemically modified to include a mutagen at either the 5' end, 3' end, or internal portion so that the mutagen is proximal to the site in the gene requiring modification. Preferably, the mutagen is incorporated into the oligonucleotide during nucleotide synthesis. For example, commercially available compounds such as psoralen C2 phosphoramidite (Glen Research, Sterling, VA) are inserted into a specific location within an oligonucleotide sequence in accordance with the methods of Takasugi et al., *Proc. Natl. Acad. Sci. U.S.A.* 88:5602-5606 (1991), Gia et al., *Biochemistry* 31:11818-11822 (1992), Giovannangeli et al., *Nucleic Acids Res.* 20:4275-4281 (1992) and Giovannangeli et al., *Proc. Natl. Acad. Sci.* U.S.A. 89:8631-8635 (1992).

The mutagen may also be attached to the oligonucleotide by a covalent bond. For example, the mutagen is attached to the oligonucleotide by a linker, such as sulfo-*m*-maleimidobenzoly-*N*-hydroxysuccinimide ester (sulfo-MBS, Pierce Chemical Co., Rockford, IL) in accordance with the methods of Liu et al., *Biochem.* 18:690-697 (1979) and Kitagawa and Ailawa, *J. Biochem.* 79:233-236 (1976), both of which are incorporated by reference herein. Alternatively, the mutagen is attached to the oligonucleotide by photoactivation, which causes the mutagen, such as psoralen, to bind to the oligonucleotide.

The mutagen can be any chemical capable of causing a mutation at the desired site of the double-stranded DNA molecule. Preferably the mutation restores the normal, functional sequence of the gene, inactivates an oncogene or activates an oncogene suppressor, or alters the function or inactivates a viral gene.

The chemical mutagen can either cause the mutation spontaneously or subsequent to activation of the mutagen, such as, for example, by exposure to light.

Preferred mutagens include psoralen, which requires activation by UVA irradiation, acridine orange, which can be activated by UVA irradiation and can be effective in the absence of light, and alkylating agents, cis-platinum analogs, hematoporphyrins and hematoporphyrin derivatives, mitomycin C, radionuclides such as ¹²⁵I, ³⁵S and ³²P, and molecules that interact with radiation to become mutagenic, such as boron that interacts with neutron capture and iodine that interacts with auger electrons. In particular, acridine orange can be used to cause a frame shift mutation, useful for gene inactivation.

If necessary for activation of the mutagen, light can be delivered to cells on the surface of the body, such as skin cells, by exposure of the area requiring treatment to a conventional light source. Light can be delivered to cells within the body by fiber optics or laser by methods known to those skilled in the art. Targeted fluorogens that provide sufficient light to activate the light-activated mutagens can also provide a useful light source.

### Method of Administration

Preferably, the mutagenic oligonucleotides are dissolved in a physiologically-acceptable carrier, such as an aqueous solution or are incorporated within liposomes, and the carrier or liposomes are injected into the organism undergoing genetic manipulation, such as an animal requiring gene therapy or anti-viral therapeutics. The preferred route of injection in mammals is intravenous. It will be understood by those skilled in the art that oligonucleotides are taken up by cells and tissues in animals such as mice without special delivery methods, vehicles or solutions.

For *in vitro* research studies, a solution containing the mutagenic oligonucleotides is added directly to a solution containing the DNA molecules of interest in accordance with methods well known to those skilled in the art and described in more detail in the examples below. *In vivo* research studies are conducted by transfecting cells with plasmid DNA and incubating the mutagenic oligonucleotides in a solution such as growth medium with the transfected cells for a sufficient amount of time for entry of the oligomers into the cells and for triplex formation. The transfected cells may be in suspension or a monolayer attached to a solid phase, or may be cells within a tissue wherein the oligonucleotide is in the extracellular fluid. The cells are then irradiated to activate the psoralen to form photoadducts and consequently mutations at the targeted site.

### Methods of Use

If the target gene contains a mutation that is the cause of a genetic disorder, then the mutagenic oligonucleotide is useful for mutagenic repair that may restore the DNA sequence of the target gene to normal. For example, the mutagenic oligonucleotide may be useful for mutagenic repair of a defective gene such as the human β-hemoglobin gene in sickle cell anemia, thalassemia and other hemoglobinopathies. If the target gene is an oncogene causing unregulated proliferation, such as in a cancer cell, then the mutagenic oligonucleotide is useful for causing a mutation that inactivates the gene and terminates or reduces the uncontrolled proliferation of the cell. The mutagenic oligonucleotide is also a useful anti-cancer agent for activating a repressor gene that has lost its ability to repress proliferation. Furthermore, the mutagenic oligonucleotide is useful as an antiviral agent when the oligonucleotide is specific for a portion of a viral genome necessary for proper proliferation or function of the virus.

The mutagenic triplex-forming oligonucleotide can also be used as a molecular biology research tool to cause site-directed mutagenesis in any gene for the study of mechanisms such as, for example, DNA repair. The oligonucleotide may also be used to study DNA repair by delivering an adduct to the DNA and studying how the adduct is processed into a mutation under various experimental conditions.

The mutagenic triplex-forming oligonucleotides will be further understood in view of the following non-limiting examples.

### Example 1: Site-specific, Targeted Mutagenesis of the supF gene of the Lambda Phage Genome

A triplex-forming oligonucleotide linked to psoralen at its 5' end was used to achieve site-specific, targeted mutagenesis in a specific gene in an intact, double-stranded lambda phage genome. Psoralen-linked oligonucleotides were obtained from either Oligos Etc. (Wilsonville, OR) or M. Talmor (Yale University, New Haven, CT) with materials from Glen Research (Sterling, VA). The psoralen was incorporated in the oligonucleotide synthesis as a psoralen phosphoramidite in accordance with the instructions provided by supplier.

As shown schematically in Figure 3, site-specific triplex formation was designed to deliver the psoralen to the targeted site in the lambda DNA, and UVA irradiation was used to activate the psoralen to form adducts and thereby induce mutations at that site. Sequence analysis of mutations in the target gene showed that almost all were in the targeted region, and 56% were found to be the same T:A to A:T transversion at the targeted base pair. The ratio of targeted to non-targeted mutagenesis was estimated by simultaneous analysis of mutagenesis in a non-targeted gene within the lambda genome, along with analysis of mutagenesis induced by a non-triplex forming (but psoralen linked) oligonucleotide. It was found that targeted mutations were produced at a frequency at least 500-fold greater than that of non-targeted mutations.

The target gene chosen was supF, an E. coli amber suppressor tyrosine tRNA gene, contained within the genome of a lambda phage vector, lambda supF as shown in Fig. 1. A 10 base homopurine oligonucleotide AG10 (5' AGGAAGGGGG 3') capable of forming a triple strand at positions 167-176 in the supF gene was identified. The ability of AG10 to bind to the supF gene was demonstrated using ³²P-labeled AG10 in an in vitro binding reaction with a 250 bp fragment containing the entire supF gene.

To demonstrate targeted, site-specific triplex formation as a prelude to mutagenesis studies, binding assays were carried out for 2 hours at 37°C in 10% sucrose, 20 mM MgCl₂, 10 mM Tris (pH 8.0), and 1 mM spermidine in a 10 µl volume. The 250 bp supF target was generated from lambda supF using the polymerase chain reaction. Each oligo (200 ng) was labelled with 50 µCi of gamma-³²P-ATP (Amersham, Arlington Heights, IL) and separated from unreacted gamma³²P-ATP by passage through a G-25 spin column (Boehringer Mannheim, Indianapolis, IN). The concentration of oligomer in the reaction mixture was 6 x 10⁻⁸ M and the oligomer:supF ratio was approximately 1:1 on a molar basis. When present, competitors were used at 200-fold molar excess.

Following the 2 hour binding step, reaction mixtures were run on a 4% acrylamide gel in 90 mM Tris base, 90 mM boric acid, 20 mM MgCl₂ with a 20% acrylamide plug. A 100 bp ladder (BRL, Bethesda, MD) was end-labelled as described for oligomers and run on gels as a size reference. Following a 4 hour run at constant voltage (150 V), the gel was visualized by autoradiography for 1 hour using Kodak X-AR film. The electrophoretic gel showed binding of the triplex forming oligonucleotide "AG10" to the supF gene target. To assay for triplex formation, ³²P-labelled oligonucleotides, either AG10 (^{5'}AGGAAGGGGG^{3'}) or the reverse sequence oligomer (GA10), were incubated with a 240 bp double-stranded fragment containing the entire supF gene. The products of the binding reactions were visualized by polyacrylamide gel electrophoresis and autoradiography.

In the electrophoretic gel, binding of labelled AG10 to the added supF DNA (lane 2) was demonstrated by the new band migrating at the position appropriate to the 250 bp supF fragment. When no supF target DNA was present, there was no band observed at this position (lane 1). Excess unlabelled AG10 competed with the ³²P-labelled AG10 (lane 3), whereas an excess of the reverse sequence oligomer (GA10, 5' GGGGGAAGGA 3') did not compete with AG10 (lane 4). In lanes 5-8, no binding of the ³²P-labelled GA10 to supF was detected: (lane 5) GA10 alone without supF; (lane 6) GA10 plus supF; (lane 7) GA10 plus supF with excess unlabelled GA10; (lane 8) GA10 plus supF with excess AG10. The reverse sequence oligomer, GA10, failed to bind to supF or to compete with AG10 for binding. AG10 linked to 4'hydroxymethyl-4,5',8-trimethylpsoralen via a 2 carbon linker arm (pso-AG10) formed a covalent bond to labeled duplex supF DNA following UVA irradiation, whereas the reverse oligomer (pso-GA10) did not.

Targeted mutagenesis was achieved by incubating pso-AG10 with lambda supF DNA in vitro to form triplex at positions 167 to 176 of the supF gene and bring the tethered psoralen into proximity with the targeted base pair at position 167 as shown in Table 1. The numbers in the table represent the frequency of mutations seen in either the supF gene or the cI gene in the lambda supF genome following the indicated treatment. The lambda DNA at 3 nM was incubated with or without a 1000-fold molar excess of the indicated oligonucleotides (3 µM).

UVA (365 nm) irradiation of selected samples was performed at a dose of 1.8 J/cm². A radiometer was used to measure lamp output (typical UVA irradiance of 5-7 mW/cm² at 320-400 nm). The DNA was packaged in vitro into phage particles, using the method of Hohn, *Methods in Enzymology* 68:299-309 (1979), and the phage particles were adsorbed to E. coli and grown as individual plaques to allow genetic analyses of the supF and cI genes. AG10 bound specifically to the supF gene, whereas the reverse sequence GA10 did not bind.

CT8 (row 3), complementary to the 3' eight nucleotides of AG10, was preincubated with psoralen-AG10 for 30 min at a 1:1 ratio to form duplex DNA and partially inhibit the ability to psoralen-AG10 to form triplex at the targeted site in the supF gene.

Photoactivation of the psoralen generated a DNA adduct, and in vitro packaging of the psoralen-AGl0-lambda suPF DNA complex allowed growth of the phage in bacteria to fix the adduct into a mutation. The phage particles were grown as individual plaques on a bacterial lawn to detect targeted mutagenesis in the supF gene and to measure the extent of non-targeted mutagenesis by screening for the function of an unrelated gene, the lambda repressor (cI) gene. Mutations in these genes yield colorless plaques among blue ones and clear plaques among turbid ones, respectively.

Pso-AG10 plus UVA treatment of the lambda DNA resulted in a mutation frequency of 0.233% in supF but approximately 100-fold less, 0.0024%, in cI. The specificity of the targeted mutagenesis is most likely even greater than this 100-fold difference, perhaps as much as 500-fold, considering that cI (765 bp) is a bigger target for mutagenesis than supF (184 bp) and the percentage of base pairs in the two genes at which mutations are detectable was similar. This difference in target size was demonstrated by the 5-fold difference in supF versus cI mutants induced by the reverse oligomer, pso-GA10. In addition, the reverse oligomer gave a 582-fold lower frequency of supF mutations (0.0004%) than did pso-AG10, but yielded a similar frequency of cI mutations. In fact, mutagenesis by the reverse oligomer was barely above background (untreated lambda DNA).

To partially inhibit formation of the triplex, an 8 base oligomer (CT8) complementary to 8 of the 10 bases of AG10 (5' CCCCCTTC 3') was preincubated at a 1:1 ratio with pso-AG10 to form a double-stranded complex. When this pre-formed complex was incubated with lambda supF and irradiated with UVA, it yielded only 0.016% supF mutations, 15-fold less than with psoralen-AG10 alone. No significant mutagenesis was produced by UVA alone (1.8 J/cm²) in the absence of the pso-AG10 or by pso-AG10 without UVA, demonstrating the importance of activation of the psoralen by UVA and showing that triplex formation, by itself, was not mutagenic. This data provided genetic evidence for the targeted mutagenesis of the supF gene by pso-AG10.

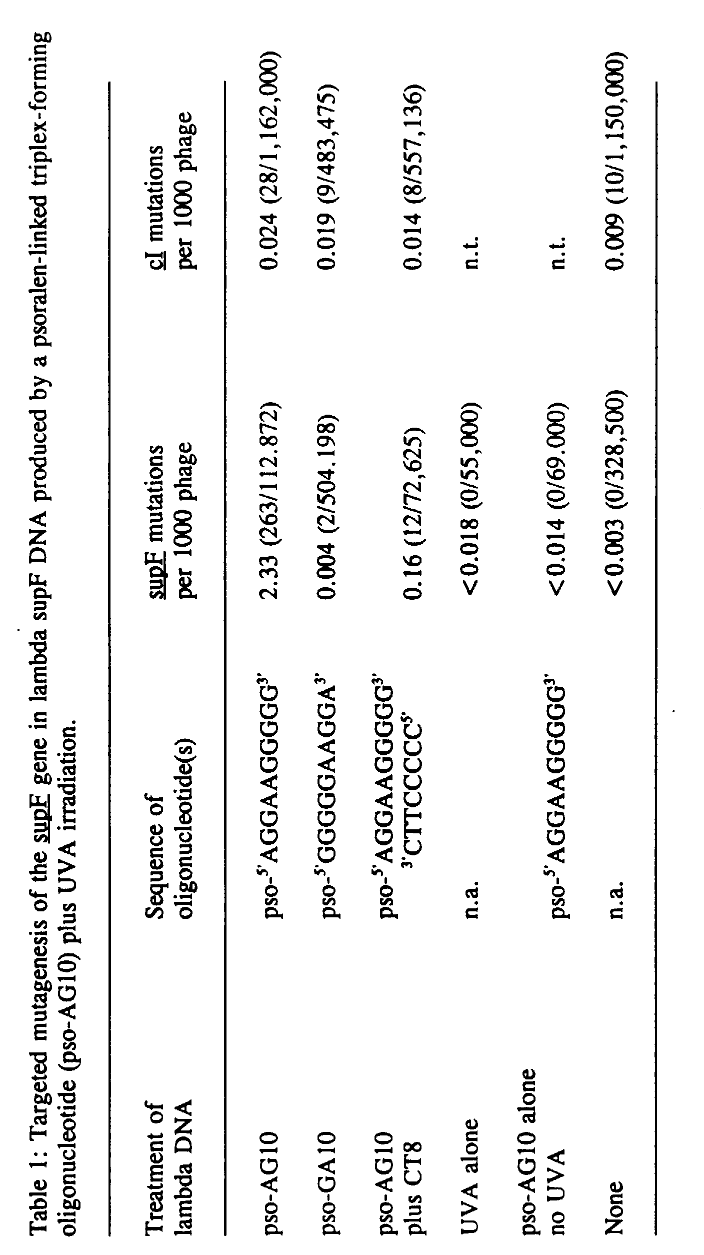

### Example 2: Sequence Analysis of Mutants Obtained by Targeted Mutagenesis Using the Triplex-Forming Oligaonucleotide

To obtain direct evidence for targeted mutagenesis, a series of independent mutants produced in the supF gene of the lambda vector by pso-AG10 and UVA were sequenced. The sequences of 25 such mutants are presented in Fig. 2a. All except one of the 25 mutations produced by pso-AG10 is at or near the targeted T:A base pair at position 167. 56% of the mutations consist of the same T:A to A:T transversion precisely at the targeted base pair (#167), demonstrating the specificity and reproducibility of the targeting by pso-AG10. The A:T base pair at 167 forms a triplet with the 5' adenine to which the psoralen is tethered in AG10, and so it is the closest base pair to the psoralen. The overwhelming predominance of the T:A to A:T transversion at this site is consistent with the mutagenic action of psoralen, which tends to form adducts at pyrimidines, and especially at thymidines. It should be noted that these mutations are independent and none of the mutations represent siblings because each packaged lambda particle gives rise to a single, separate lambda plaque on the bacterial lawn.

Mutations were found to be induced in the supF gene by free 8-methoxypsoralen and UVA in other experimental systems employing shuttle vectors, as described by Glazer et al., *Proc. Natl. Acad. Sci.* USA 83:1041 (1986) and Bredberg and Nachmansson, *Carcinogenesis* 8:1923 (1987). This compiled data demonstrates that free psoralen can form adducts and induce mutations at many different sites in supF apart from base pair 167. The scattered distribution of mutations is in contrast with the specific mutagenesis induced by the triplex-forming pso-AG10. Although several of the mutations listed in Fig. 2b fall in the region of the homopurine/homopyrimidine run at positions 167 to 176, none of them occur at position 167. Neither of the two mutations induced by the reverse oligomer, pso-GA10, were found to occur at base pair 167.

The spectrum of the mutations produced by pso-AG10 indicates that almost all were targeted by the triplex-forming oligonucleotide. Although a majority of the mutations were at the targeted position 167 and consisted of the same T:A to A:T transversion, several mutations were at base pairs nearby to position 167. It is possible that the psoralen moiety, tethered to AG10 on a 2 carbon linker arm, may occasionally reach beyond the T:A base pair at 167 to form adducts at nearby pyrimidines, giving rise to mutations. It is also possible that even if an adduct is formed at position 167, the bacterial polymerase and repair enzymes that fix the adduct into a mutation may generate mutations at nearby sites during repair and replication while at the same time repairing or bypassing the adduct at 167. The occurrence of several mutations that involve base changes at two adjacent base pairs (166 and 167 in all 3 instances) supports the notion that an adduct at position 167 can cause a change at a nearby position. The rare non-specific mutagenesis by pso-AG10 (and the very small amount of mutagenesis by pso-GA10 that is above background) may result from the potential ability of the psoralen molecule, in spite of being tethered to the oligonucleotide, to intercalate into and form adducts at random sites in the DNA. A reduction of this non-specific activity may be achieved by reducing the reach and the degrees of freedom of the psoralen by attaching it to the triplex-forming oligonucleotide by a shorter tether, such as a one carbon linker arm, or by direct linkage of the psoralen to the nucleotide in the triplex-forming oligonucleotide by direct photoactivation of free psoralen to bind to the oligonucleotide, and the purification of the desired product.

This experiment achieved a targeted mutation frequency of 0.233%.

### Example 3: Covalent Linkage of Psoralen to an Oligonucleotide.

The mutagen, 5-aminomethyl-8-methoxypsoralen, was covalently linked to an oligonucleotide.

5-aminomethyl-8-methoxypsoralen (5am8mop, HRI Associates, Emeryville, CA) was mixed with the linker, sulfo-m-maleimidobenzoyl-N-hydroxysuccinimide ester (sulfo-MBS, Pierce Chemical Co., Rockville, IL) in 0.05 M phosphate buffer, pH 8, with a 5am8mop to sulfo-MBS molar ratio of 1:40. The mixture was stirred at room temperature for 30 minutes while protected from light in accordance with the methods of Liu et al., *Biochem.* 18:690-697 (1979) and Kitagawa and Ailawa, *J. Biochem.* 79:233-236 (1976), and the instructions of the Pierce Immunotechnology Catalog and Handbook, 1992-93 edition, pages A16-A17. The initial run was made using 1 ml total volume and 1 mM 5am8mop, with the reaction scaled up and optimized as needed.

The modified 5am8mop was purified by HPLC using a modification of standard conditions used in the analysis of 8-methoxypsoralen as described by Gasparro et al., *J. Invest. Derm.* 90:234-236 (1988). The initial conditions were: a Regis Rexchrom™ phenyl 15 cm HPLC column running a gradient between acetonitrile and either water or 0.05 M, pH 4.5 ammonium acetate buffer. A linear gradient was run from 10% acetonitrile to 60% acetonitrile over 50 minutes. When buffer was needed in the initial purification run, the sample was collected off the HPLC, evaporated, and desalted by passing it through the HPLC again with an acetonitrile:water gradient mixture. The detector was a SpectraFocus^{™} scanning UV detector with wavelengths from 220 to 360 sampled. The detector was connected to a Pharmacia Frac-100™ fraction collector.

The purified, modified 5am8mop was then reacted with an oligonucleotide containing an -SH tether by mixing equimolar amounts of modified 5am8mop with the oligonucleotide in 0.05 M phosphate buffer, pH 7-7.5 at room temperature for three hours while protected from light.

The oligonucleotide tethered to 5am8mop was then purified by HPLC using a modification of the method of Gasparro et al., *Antisense Res. Dev.* 1:117-140 (1991). A Nest Group MRPH 10 cm HPLC column running a linear gradient of 5% to 20% acetonitrile over 40 minutes between acetonitrile and 0.2 M pH 5.9 triethylammonium acetate buffer was used.

### Example 4: Targeted Mutagenesis of SV40 DNA Using Triple Helix-Forming Oligonucleotides.

The following was performed to investigate targeted mutagenesis of SV40 DNA transfected into monkey cells. In these experiments, the site-specific triplex formation was designed to deliver the psoralen to the targeted site in the SV40 DNA, UVA irradiation was used to activate the psoralen to form adducts at that site, and repair and replication of the viral genomes in the monkey cells fix the adducts into mutations. These results demonstrate that targeted mutagenesis occurs even more efficiently in mammalian cells (6% of SV40 genomes incurred targeted mutations) than in bacteria (0.2%).

### MATERIALS AND METHODS

**Oligonucleotides and vectors.** Psoralen-linked oligonucleotides were obtained from either Oligos Etc. (Wilsonville, OR) or M. Talmor (Yale University, New Haven, CT) with materials from Glen Research (Sterling, VA). The psoralen is incorporated in the oligonucleotide synthesis as a psoralen phosphoramidite, resulting in an oligonucleotide linked at its 5' end via a two carbon linker arm to 4'-hydroxymethyl-4,5',8-trimethylpsoralen, as illustrated in Fig. 3. The sequences of oligonucleotides used in this study include AG10 (5'AGGAAGGGGG3') and GA10 (5'GGGGGAAGGA3'). SV40 shuttle vector pSP189 was constructed by and obtained from Dr. Michael Seidman (Otsuka Pharmaceuticals, Bethesda, MD). **Triplex binding assays.** Binding assays were carried out for 2 hours at 37°C in 10% sucrose, 20 mM MgCl₂, 10 mM Tris (pH 8.0), and 1 mM spermidine in a 10 µl volume. The 250 bp supF target was generated from lambda supF using the polymerase chain reaction.
**Protection assay using PCR amplified supF target.** The 250 bp supF target (70 nM) was incubated with a 100-fold molar excess of psoralen-AG10 as described for the binding assay. Irradiation of samples was performed at a dose of 1.8 J/cm². A radiometer was used to measure the lamp output (typical UVA irradiance of 5-7 mW/cm² at 320-400 nm). Following the binding and irradiation steps, samples were digested for 2 hours at 37°C with *Hin*f I. Loading buffer was added and samples were heated 10 minutes at 55°C, and run for 1 hour on a 4.5% Nusieve gel in TAE buffer at 80 v (10 v/cm). An analysis by agarose gel electrophoresis of *Hin*f I digestions of the 250 bp supF gene PCR fragment under various conditions was performed. A faint band corresponding to a size of 150 kDa was present in Lane 1, which contained no psoralen-AG10 and no UVA; a band corresponding to a size of 150 kDa was present in lane 2, containing UVA alone (no psoralen-AG10); a band corresponding to a size of 150 kDa was present in lane 3, psoralen-AG10 and UVA; a band corresponding to a size of 150 kDa was present in lane 4, psoralen-AG10 alone (no UVA); a band corresponding to a size of approximately 300 kDa was present in lane 5, undigested supF PCR fragment; lane 6 contained the size markers (100 bp ladder) and bands were present at 100, 200 and 300 kDa.
**Protection assay using SV40 vector DNA target.** The binding and irradiation were carried out as described above, except that pSP189 was used as the supF target at a concentration of 50 nM and psoralen-AG10 was added at ratios of oligomer to vector of from 1:1 to 1000:1. Irradiation and gel conditions were as described above.
**Colony hybridization.** Ampicillin resistant colonies of SY204 carrying shuttle vector plasmids with supF gene mutations, along with appropriate control colonies, were grown on LB/ampicillin plates and transferred onto replica nylon filters for additional growth and in *situ* lysis to allow colony hybridization by standard methods. The DNA was fixed to the filters by UV crosslinking, and the filters were incubated in 6X SSC, 5X Denhardt's solution, 0.5% SDS, and 5 x 10⁵ cpm/ml of ³²P-labeled oligonucleotides at 42°C for 18 hours. The filters were washed in 1X SSC and 0.1% SDS for 30 minutes at 25°C and then in 1X SSC and 0.1% SDS at 42°C for 2 hours. These conditions were empirically determined to allow discrimination between binding of the wild type probe (5' GGT TCG AAT CCT TCC CCC 3') and the 167 mutant probe (5' GGT TCG AAA CCT TCC CCC 3'). Binding of the oligonucleotide probes was determined by autoradiography.
**SV40 mutagenesis.** The SV40 vector DNA (pSP189) at 80 nM was incubated with psoralen-AG10 or psoralen-GA10 (ranging from 2 to 1000-fold molar excess) and irradiated as described above. The oligonucleotide-plasmid complex was then transfected into monkey COS-7 cells (ATCC #1651-CRL) using cationic liposomes (DOTAP, Boehringer Mannheim, Indianapolis, IN) at a final concentration of 5 µg/ml in the culture dish. The DNA/oligo/liposome mixture was added dropwise to the cell culture dish with swirling. The following day, the media containing the liposome mixture was replaced by fresh media. Following 48 hours to allow repair and replication, SV40 vector DNA was harvested from the COS cells by the Hirt lysate procedure. Genetic analysis of the supF genes in the SV40 vector was carried out by transformation of E. coli SY204 [*lacZ*l25(Am)] to ampicillin resistance by electroporation using 12-150 ng of *Dpn* I digested Hirt lysate DNA and a Bio-Rad Gene Pulser apparatus equipped with a Pulse Controller (Bio-Rad, Richmond, CA). Mutants were identified by growth in the presence of 65 µg/ml IPTG and 80 µg/ml X-Gal, as described by (Glazer et al., *Mol. Cell Biol.* 7:218-224 (1987)). These transformants were counted and the mutants (white colonies) were streaked for single colonies.
**DNA sequencing.** DNA was prepared for sequencing by isolating DNA from a 3 ml bacterial culture using a Promega Magic Miniprep kit (Promega, Madison, WI). DNA sequence data was obtained by direct chain termination sequencing of the plasmid DNA using automated methods.

### RESULTS

Strategy for targeted mutagenesis in SV40. An SV40-based shuttle vector (pS189) was used to assay for targeted mutagenesis. This vector contains both the SV40 and the pBR328 origins of replication, plus the β-lactamase gene for ampicillin resistance, to allow episomal replication in both mammalian cells and bacteria (Fig. 3). It also carries the supF gene, an amber suppressor tyrosine tRNA gene of E. coli, as a marker gene for mutagenesis studies.

In this vector system, the SV40 DNA, after appropriate treatment, is introduced into monkey COS cells where repair and replication can occur, producing mutations indicative of mammalian processing of DNA damage. The small, circular vector DNA is recovered from the cells by biochemical separation from the chromosomal DNA (Hirt lysate, Hirt et al., J. *Mol. Biol.* 26:365-369 (1967)), and it is used to transform E. coli carrying the lacZ (amber) mutation to allow analysis of supF gene function by scoring colonies for β-galactosidase activity (produced via suppression of the amber mutation in lacz) in the presence of the chromogenic substrate, X-gal. Vectors with wild type supF genes yield blue colonies; those with mutations in supF produce white ones. In order to eliminate misleading data that might arise from viral DNA that was not replicated or repaired in the mammalian cells, the viral DNA is digested before bacterial transformation with the enzyme *Dpn* I which will restrict DNA that has not been methylated by the mammalian pattern at its recognition site.

The design of the initial experiments to target mutations to SV40 DNA is illustrated in Fig. 3. A 10 base pair region of the supF gene (bp 167-176) was identified as a site amenable to triplex formation because of the homopurine/homopyrimidine run there. Since this run was G-rich, the purine motif for triplex formation was selected (Beal and Dervan, *Science* 251:1360-1363 (1991)), and an oligonucleotide, 5'AGGAAGGGGG3' (AG10) was synthesized based on this motif. A psoralen derivative, 4'-hydroxymethyl-4,5',8-trimethylpsoralen, was attached to the oligonucleotide by a phosphodiester linkage at the 5' adenine via a two carbon linker arm, with the goal of directing mutations to base pair 167. This is the base pair with which that 5' adenine binds in the predicted triple helix. Note that the psoralen-AG10 oligonucleotide is oriented anti-parallel to the purine-rich strand in the duplex DNA. To achieve targeted mutagenesis, the pSP189 DNA is incubated with the psoralen-linked oligonucleotide (psoralen-AG10), treated with long wave ultraviolet light (UVA) to activate the psoralen to form a pre-mutagenic adduct on the thymidine in base pair 167, and then transfected into COS-7 cells. After a 48 hour period to allow repair and replication, the viral DNA is isolated from the monkey cells, subjected to digestion with *Dpn* I, and used to transform E. coli. The frequency of supF mutations is determined, and representative samples of supF mutant clones are collected for further analysis.
**Site-specific formation of triplex DNA.** This experiment demonstrate the ability of psoralen-AG10 to bind specifically to the intended site within the supF gene using a restriction enzyme protection assay. In this assay, psoralen-AG10 was found to bind site-specifically to duplex supF DNA following UVA irradiation, blocking restriction enzyme digestion at the one *Hin*f I site (bp 164-168) that overlaps the triplex target site (167-176) but not at the other *Hin*f I site in supF (bp 129-133).
This is diagrammed in Fig. 4 and was demonstrated by the electrophoretic gel (not shown) which showed site-specific formation of triplex DNA in the SV40 vector as a function of the ratio of oligonucleotide to SV40 DNA. The SV40 vector containing the supF target gene (50 nM) was incubated with psoralen-AG10 at ratios of oligomer to vector of from 1:1 to 1000:1, irradiated with 1.8 J/cm² of UVA, digested with *Hin*f I, and run on a 4.5% Nusieve gel. Lane 1, undigested plasmid DNA; lane 2, no psoralen-AG10 prior to digestion; lanes 3-7, increasing ratios of psoralen-AG10/SV40 DNA as indicated above each lane; lane 8, 100 bp size markers (BRL-Gibco; a trade mark). Because the sequences flanking the supF gene in the SV40 DNA differ from those in the PCR fragment presented in the gel described above and since there are multiple *Hin*f I sites in SV40, the pattern of bands is more complex than those in the gel described above. However, this gel indicates the position of the fragment resulting from shielding of the *Hin*f I site at bp 164-168 by triplex formation with a band between size markers 100 and 200.

Digestion of the unprotected 250 bp supF PCR fragment with *Hin*f I yields three fragments of sizes 150, 65, and 35 (lane 1), in contrast with the uncut fragment of 250 bp (lane 6). Incubation of the supF fragment with psoralen-AG10 along with photoactivation with UVA (lane 3) results in protection of the *Hin*f I site at bp 164-168 but not the one at bp 129-133, as demonstrated by the appearance of the 100 bp fragment instead of the 65 bp and 35 bp fragments. UVA-induced covalent adduct formation is required for restriction enzyme protection, since psoralen-AG10 alone is not sufficient to prevent *Hin*f I digestion (lane 4). In the absence of psoralen-AG10, UVA light had no effect on *Hin*f I digestion (lane 2). In similar experiments, no protection from *Hin*f I cutting was seen using psoralen-GA10, the reverse sequence oligomer linked to psoralen. This data demonstrates site-specific formation of triplex DNA by psoralen-AG10, with covalent modification of the supF gene fragment occurring at the targeted site following UVA irradiation of the psoralen-AG10/supf complex.

Similar experiments were performed to assay for site-specific binding of psoralen-AG10 to bp 167-176 in the supF gene within the SV40 vector itself. In these experiments, varying ratios of oligonucleotide to vector DNA were employed in order to examine basic parameters of the triplex binding to the viral genome. The above described gel indicates that *Hin*f I protection at the targeted site is almost complete at a 10:1 ratio of oligonucleotide to vector, as judged by the appearance in the ethidium bromide stained agarose gel of a band at 125 bp (arrow) and the disappearance of the band at 90 bp. Ratios of 100:1 and 1000:1 similarly yielded near complete protection, whereas the lower ratios of 1:1 and 2:1 gave only partial protection. These results are consistent with the mutagenesis experiments, described below.

**Targeted mutagenesis of SV40 vector DNA passaged in COS cells.** Experiments to induce targeted mutagenesis in SV40-vector DNA using triplex-forming oligonucleotides were carried out as shown in Fig. 3. Psoralen-linked oligonucleotides were incubated with SV40 vector DNA, exposed to 1.8 J/m² UVA light, and transfected into COS cells. After two days to allow repair and replication to occur, the vector DNA was rescued from the cells and used to transform bacteria to facilitate genetic analysis of the supF gene. The effect of psoralen-AG10, which binds site-specifically to the supF gene in the vector, in inducing supF mutations was compared to that of psoralen-GA10, which shows no specific binding. Various ratios of oligonucleotide to vector DNA were used in order to investigate parameters that might affect the specificity and the efficiency of the process of targeted mutagenesis in the monkey cells. Table 2 presents the data from these experiments. Targeted mutations in the supF gene were produced in the SV40 genome at a frequency as high as 7.3 % using psoralen-AG10 at a molar ratio of oligonucleotide to vector DNA of 1000 to 1. At this same ratio, psoralen-GA10 produced a small amount of mutagenesis above background (0.5% versus 0.07%). At the lower ratios tested, however, the reverse oligomer yielded no significant mutagenesis above the background frequency in the assay, whereas, at these lower ratios, psoralen-AG10 still generated a high frequency of mutations in supF (as high as 6.4% for the 10:1 ratio versus 0.06% for psoralen-GA10 at 10:1 and 0.07% for untreated vector DNA). This demonstrates mutagenesis specifically targeted to the supF gene in the SV40 vector by psoralen-AG10 but not by psoralen-GA10. This frequency of targeted mutagenesis in SV40, in the range of 6% to 7%, is 30-fold higher than that seen in previous experiments to target the supF gene in bacteriophage lambda grown in E. coli (0.23 %, Havre et al., *Proc. Natl. Acad. Sci.* 90:7879-7883 (1993)), and it suggests that the monkey cells more efficiently process the pre-mutagenic lesion of the psoralen/oligonucleotide adduct into a mutation, via either error-prone repair or bypass replication.

In control experiments (Table 2), UVA irradiation of the SV40 DNA, in the absence of the psoralen-linked oligonucleotides, produced no mutagenesis above background. Similarly, the treatment of the SV40 DNA with the oligomers but without UVA irradiation was not mutagenic.

**Sequence analysis of targeted mutations.** A set of 20 mutants generated in the supF gene in the SV40 vector by psoralen-AG10 (at the 1000:1 ratio) and UVA light were subjected to DNA sequence analysis. The results of this analysis are shown in Fig. 5A. Of the 20 mutations analyzed, 11 consist of the same T:A to A:T transversion at base pair 167 occurring over and over again. This is the precise base pair to which the mutations were targeted by psoralen-AG10, as diagrammed in Figs. 5A and 5B. The finding that 55% of the sequenced mutations consisted of the exact same base change at the targeted base pair suggests that the intended base change (T:A to A:T at bp 167) was produced in over 4% of all the viral genomes. The other mutations analyzed included 3 point mutations at base pairs adjacent to the targeted base pair and 6 small deletions including or abutting that base pair. These likely arise from variations in the processing, repair, or replicative by-pass of the triplex-directed lesion at bp 167 as the SV40 DNA is replicated in the monkey cells. It is also possible that the psoralen molecule, tethered to the oligonucleotide by a 2-carbon linker arm, has sufficient reach and degrees of freedom to form adducts at nearby base pairs. Improved mutational specificity may be achieved by reducing the length of the linker arm. In Fig. 5B, the published sequences of supF mutations produced in this same vector system using free 8-methoxypsoralen are presented for comparison. Not only are these mutations more scattered, but also none were found to occur at base pair 167.

In the analysis of mutagenesis in SV40 vectors, it is often difficult to determine if identical mutations arose independently or if they were the result of a single mutational event amplified by subsequent vector replication. In order to exclude the possibility that such sibling mutations were isolated in these experiments, use was made of an advantageous feature of the pSP189 system, in which over 100,000 different, random-sequence 8 base pair oligonucleotides were cloned into a region of pSP189 next to the supF gene. The vector DNA is prepared *en masse* from this library of vector clones containing the different 8 bp sequences. In this way, at the same time that the sequence of the supF gene in a mutant vector is ascertained, the 8 base pair signature sequence in that particular plasmid molecule can also be identified by reading a few extra bases further in the sequence data. This enables comparisons between the 8 bp signature sequences in plasmids bearing the same supF mutation to see if they are siblings from the same mutational event or if they are independent mutations. Based on this analysis, it was determined that all 20 of the mutations presented here arose independently.

In order to strengthen and confirm these results, a larger sample of supF mutations produced in the SV40 vector by psoralen-AG10 and UVA light was analyzed by an alternate method based on the expected high proportion of T:A to A:T transversions at bp 167. Instead of direct sequencing, a technique of differential oligonucleotide hybridizations was used (Sidransky et al., *Science* 252:706-709 (1991)). In this assay, undertaken in an effort to streamline mutant analysis, ampicillin resistant bacterial colonies containing mutant supF genes were grown on nylon filters to allow nucleic acid hybridizations. Duplicate filters were incubated with ³²P-labelled, 18 base oligonucleotides that either matched the wild type sequence or matched the position 167 T:A to A:T mutant sequence. The hybridizations were carried out by standard methods under conditions empirically determined to be stringent enough to allow differentiation between mutant and wild type sequences. Of the 19 colonies assayed in this particular experiment, 9 showed hybridization specific to the mutant probe. None showed hybridization to the wild type probe, except for the positive control in the upper right corner. For the 9 colonies that bind to the 167 probe, this supports the validity of the assay. For the other 10 that did not bind to the mutant probe either, the lack of binding to the wild type probe suggests that they either have different mutations at bp 167 (not T:A to A:T) or have mutations near bp 167, within the 18 bp region covered by the probes, causing mismatches with both the wild type and mutant oligonucleotides. A total of 42 mutants generated by psoralen-AG10 were analyzed by this method (including the 20 subject to sequence analysis), and 22 (52%) were found to carry the T:A to A:T mutation at bp 167. All of the rest were judged to have different mutations at or near the targeted base pair, because neither the mutant nor wild type probe hybridized to them. The validity of this assay was supported by the 100% agreement with the sequencing data. These results extend the direct sequencing data and demonstrate further the targeted mutagenesis of SV40 vector DNA. Taken together, the data suggests that almost all of the mutations produced by psoralen-AG10 are at or within a few bases of the targeted base pair, and at least 50% consist of the same T:A to A:T transversion at that site. These results demonstrate efficient production of specific, reproducible, and predictable mutations at a targeted base pair in SV40 DNA passaged in monkey cells.

**TABLE 2.**

| Targeted mutagenesis in SV40 DNA | | | |
|---|---|---|---|
| Treatment of SV40 vector DNA^{*a*} | Ratio of oligo to vector | % mutants^{*b*} | Mutants per total colonies |
| None | n.a. | 0.07 | 6 / 8,190 |
| | | | |
| psoralen-AG10^{c} no UVA | 1000:1 | ≤0.06 | 0 / 1700 |
| | | | |
| psoralen-GA10^{c} no UVA | 1000:1 | ≤ 0.07 | 0 / 1500 |
| | | | |
| UVA alone | n.a. | 0.06 | 5 / 8,427 |
| | | | |
| psoralen-AG10^{*c*} | 2:1 | 2.5 | 148 / 5,869 |
| | 5:1 | 4.3 | 118/ 2,734 |
| | 10:1 | 6.4 | 381 / 5,995 |
| | 1000:1 | 7.3 | 633 / 8,643 |
| | | | |
| psoralen-GA10^{c} | 2:1 | 0.07 | 3 / 4,397 |
| | 5:1 | 0.13 | 11 /8 ,230 |
| | 10:1 | 0.06 | 4 / 6,800 |
| | 1000:1 | 0.63 | 92 / 14,670 |
| | | | |

| | | | |
|---|---|---|---|
| ^{*a*} Except where indicated, all samples received 1.8 J/cm² of UVA irradiation. | | | |
| ^{*b*} The values represent the frequency of mutations seen in the supF gene within the pSP189 SV40 vector. | | | |
| ^{*c*} Psoralen-AG10 forms a site-specific triple strand at bp 167-176 of the supF gene within pSP189; the reverse sequence oligomer, psoralen-GA10, does not. | | | |

### Example 5: Intracellular Targeted Mutagenesis.

An experiment demonstrating targeted mutagenesis of an SV40 vector mediated by intracellular triple helix formation in monkey COS cells was performed. The results demonstrated that specific, reproducible mutations can be produced in viral genomes replicating in monkey cells by treatment of the cells with psoralen-linked oligonucleotides followed by photoactivation of the psoralen with long wavelength UV light (UVA). Using a set of modified target sites in the *supF* gene and a series of oligonucleotides, this experiment demonstrated that targeted mutagenesis in an in *vivo* assay depends on the specificity of the mutagenic oligonucleotide for the target site and on the strength of the oligonucleotide binding. The results also provide a preliminary analysis of both the time dependence and the concentration dependence of the oligonucleotide-directed targeting within mammalian cells.

### Materials and Methods.

**Oligonucleotides and vectors.** Psoralen-linked oligonucleotides were obtained from Oligos Etc. (Wilsonville, OR). The psoralen was incorporated into the oligonucleotide synthesis as a psoralen phosphoramidite, resulting in an oligonucleotide linked at its 5' end via a two-carbon linker arm to 4'-hydroxymethyl-4',5'.8-trimethylpsoralen, as illustrated in Fig. 6. The sequences of psoralen-conjugated oligonucleotides used in this experiment include:

AG10   5′ AGGAAGGGG 3′
AGT20  5′ AGGAAGGGGGGGTGGTGGG 3′
AGT30  5′ AGGAAGGGGGGGTGGTGGGGAGGGGGAG 3′
AGT43  5′ AGGAAGGGGGGGTGGTGGGGAGGGGG
          AGGGGGAGGGGGAGG 3′

SV40 shuttle vectors, pSupFGla and pSupFG2 were derivatives of pSP189 (described above) and carried new triplex-binding sites which were engineered into the *supF* gene. The modified *supF* genes were constructed by inserting synthetic oligonucleotides into the *Xho*I to *Eag*I sites in the original *supF* gene using standard techniques as described by Sambrook et al., MOLECULAR CLONING: A LABORATORY MANUAL, second edition, Cold Spring Harbor Laboratory Press, New York (1990).

**Triplex binding assays.** Two complementary 57-mere which contain the sequence corresponding to bp 157 to 213 of *supFG1a* were synthesized. Both oligomers were labeled with y-[³²P]-ATP. The duplex DNA was prepared by mixing both 57-mere at a ratio of 1:1 in TE buffer and incubating at 37°C for two hours. A fixed concentration of duplex DNA (1 x 10⁻¹⁰ M) was incubated with increasing concentrations of the psoralen-linked oligomers in 10 µl of 10 mM Tris (pH 7.4), 1 mM spermidine, and 20 mM MgCl₂ at 37°C for two hours. UVA irradiation (1.8 J/cm² of broad band UV light centered at 365 nm, irradiance of 5 mW/cm²) was used to generate photoadducts and thereby covalently link the oligomers to their targets. The samples were mixed with 90 µl of formamide, and 20 µl of each sample was analyzed on an 8% polyacrylamide denaturing gel containing SDS and 7 M urea. A phosphor-imager (Molecular Dynamics, Sunnyvale, CA) was used for quantitation of the reaction products. The concentration at which triplex formation (as indicated by the generation of specific photoadducts) was half-maximal was taken as the equilibrium dissociation constant (Kd).

**Mutagenesis protocol.** Transfection of the cells with plasmid and treatment of the cells with the psoralen-oligonucleotide was performed as follows. Monkey COS-7 cells were obtained from the ATCC (1651-CRL) (Rockville, MD). The COS cells at 70% confluence were washed with PBS-EDTA, treated with trypsin and incubated at 37°C for five minutes. The cells were resuspended in DMEM/10%FCS and were washed three times by centrifugation at 900 rpm for five minutes (4°C) using a Sorvall RT6000D centrifuge. The cells were resuspended at 1 x 10⁷ cells/ml. The plasmid DNAs were added at 3 µg DNA/10⁶ cells and the cell/DNA mixtures were left on ice for 10 minutes. Transfection of the cells was performed by electroporation using a Bio-Rad gene pulser at a setting of 25 µF/250 W/250 V in the 0.4 cm cuvette. Following electroporation, the cells were kept on ice for 10 minutes. The cells were diluted with growth medium, washed, and transferred to 37°C for 30 minutes. At this point, the cells were either further diluted and exposed to the oligonucleotides in growth medium or washed, diluted further, and allowed to attach to dishes for twelve hours, washed again with PBS/EDTA, trypsinized, washed three times with growth medium, and finally exposed to the oligonucleotides in suspension. In each case, the psoralen-conjugated oligonucleotides were added to cells in suspension, which were then incubated at 37°C with gentle agitation every fifteen minutes. UVA irradiation was given at a dose of 1.8 J/cm² at the indicated times. The cells were further diluted in growth medium and allowed to attach to plastic dishes at a density of 1 x 10⁶ cells per 15 cm² dish.

After 48 hours, the cells were harvested for vector DNA isolation using a modified alkaline lysis procedure. The cells were resuspended in 100 µl of cell resuspension solution (50 mM Tris/HCl, 10 mM EDTA, pH 8.0; 100 µg/ml RNase A) and 100 µl of cell lysis solution (0.2 M NaOH, 1% SDS) was added. After five minutes at room temperature, 100 µl of neutralization solution (3 M potassium acetate, pH 5.5) was added. A fifteen minute room temperature incubation was followed by centrifugation in a microcentrifuge for ten minutes. The clear supernatant was extracted with an equal volume of phenol/chloroform (1:1) once, and the DNA was precipitated with 2.5 volumes of ethanol at -70°C for ten minutes. The DNA was collected by centrifugation for ten minutes, washed with 70% ethanol once, and allowed to air dry for five minutes at room temperature. The DNA was digested with *Dpn* I and RNase A at 37°C for two hours, extracted with phenol/chloroform, and precipitated with ethanol. The DNA pellet was dissolved in 10 µl of TE buffer (10 mM Tris, 1 mM EDTA, pH 8.0) and 1 µl of vector DNA was used to transform *E. coli* SY204 or MBM7070 by electroporation. The transformed *E. coli* cells were plated onto LB plates containing 50 µg/ml of ampicillin, 100 µg/ml of X-gal, and 1 M of IPTG and were incubated at 37°C overnight. The mutant colonies and the total colonies were counted. The mutant colonies were purified and the plasmids were isolated for DNA sequence analysis.

**DNA sequencing.** The single colonies of purified mutants were picked into 5 ml of L broth containing ampicillin (50 µg/ml) and were incubated at 37°C for 16-20 hours by shaking at 250 rpm. Cells from 3 milliliters of culture were collected by centrifugation. Isolation of plasmid DNA was accomplished using the Wizard plasmid miniprep DNA purification system (Promega, Madison, WI). 1.5 µg of plasmid DNA was used for DNA sequencing using an ABI cycle-sequencing kit in accordance with the manufacturer's instructions (Applied Biosystems Inc., Foster City, CA) using standard methods. The sequencing primer was chosen to bind to the β-lactamase gene just upstream of the *supF* gene in the vector.

### Results.

**Mutagenesis assay.** The design of the assay system used to study targeted mutagenesis of an SV40 vector *in vivo* in Monkey COS cells is shown in Fig. 6. In addition to the SV40 sequences necessary to enable replication in mammalian cells, the SV40 shuttle vector contains the *supF* gene, a suppressor tRNA gene of *E. coli,* as the target gene for mutagenesis. It also contains a portion of the pBR327 replication origin and β-lactamase gene for growth and selection in bacteria. Following introduction of the vector DNA into the COS cells by electroporation, the cells were incubated in the presence of the psoralen-linked oligonucleotides, which are designed to bind to *supF* gene sequences such that the psoralen is delivered to the intended intercalation site at bp 166-167. After allowing a variable time for entry of the oligomers into the cells and for triplex formation, the cells were irradiated with UVA to activate the psoralen to form photoadducts and consequently mutations at the targeted site in the *supF* gene. Another 48 hours were allowed for repair and/or replication. The vector DNA was harvested from the cells by an alkaline lysis procedure and used to transform *lacZ* (amber) *E. coli* to detect mutations in the *supF* gene that occurred in the COS cells. Prior to transformation, *Dpn* I digestion of the vector DNA was used to eliminate unreplicated vector molecules lacking the mammalian methylation pattern and thereby preclude misleading data resulting from unprocessed input molecules.

**Insufficient *in vivo* targeting by a 10-mer.** In the experiments described above regarding *in vitro* experiments, a 10 base pair region of the *supF* gene, bp 167-176, was targeted for triplex formation in the anti-parallel motif using a 10 nucleotide psoralen-linked oligomer, pso-AG10. In these experiments, the triplex formation was allowed to occur during a two hour incubation *in vitro* in an optimized Mg²⁺ containing buffer, followed by *in vitro* UVA irradiation and then transfection of the vector/oligonucleotide complex into the cells as described above. Mutations were generated in the target gene at a frequency of approximately 6 to 7%. In subsequent experiments, the SV40 vector and pso-AG10 were co-transfected into the cells, with UVA irradiation of the cells two hours later. This protocol yielded targeted mutations in 2.1% of the vector molecules, a frequency in the range of that observed in the above-described *in vitro* work. However, attempts to use pso-AG10 to target mutations to the *supF* gene by treatment of cells already pre-transfected with the pSP189 vector in the protocol outlined above (Fig. 6), resulted in detection of little mutagenesis (0.05%) above background (0.02%).

**Construction of novel *supF* genes.** Based on this result and based on the reported binding constants for oligonucleotides of various lengths, a modified *supF* gene containing a 30 base pair polypurine/polypyrimidine segment amenable to triplex formation was constructed because it was believed that the binding affinity for triple helix formation by the 10-mer (pso-AG10) might be insufficient to achieve significant interactions *in vivo.* Novel gene sequences were incorporated into the *supF* gene to test a series of potential triplex forming oligonucleotides using bp 166-167 as the targeted psoralen intercalation site.

Assay systems were developed to allow the characterization of factors affecting *in vivo* triplex formation and to examine the possibility of intracellular mutation targeting.

The pSP189 vector contains a 93 bp segment encompassing the sequences coding for the mature tRNA between unique *Xho*I and *Eag*I sites. Using synthetic oligonucleotides, this 93 bp stretch was replaced with novel sequences. The design of a new *supF* gene, *supFGla,* is illustrated in Fig. 7. To eliminate one interruption at bp 167 in the polypurine/polypyrimidine run, an A:T to C:G transversion was incorporated into the synthetic fragment, along with a compensatory T:A to G:C change at bp 101 to maintain base pairing in the amino acid acceptor stem of the mature tRNA. In addition, a 13 bp polypurine/polypyrimidine sequence was inserted between bp 183 and 184 to extend the length of the polypurine/polypyrimidine run in the gene to 30 bp. Since the 5' CCA sequence at positions 181-183 comprises the 3' terminal amino acid acceptor site of the tRNA, the new sequences 3' to position 183 do not affect the mature tRNA molecule and so do not alter the phenotype of the gene. Following annealing and legation of the synthetic oligonucleotides into the vector, constructs containing functional suppressor genes were identified by transformation of *lacZ* (amber) bacteria. The sequence of the new *supFGla* gene was confirmed by direct DNA sequencing of the vector DNA. The new construct contained a 30 bp polypurine site with just two interruptions. By a similar method, *supFG2* was also constructed, containing a 43 bp polypurine site, also with just two interruptions, as shown in Fig. 8.

**Triplex binding.** To compare triplex formation at the polypurine sites in *supF, supFGla,* and *supFG2,* a gel mobility shift assay was used in which synthetic DNA fragments of either 24 bp (matching bp 160-183 in the *supF* gene), 57 bp (matching bp 157-213 in *supFGla),* or 59 bp (matching bp 159-217 in *supFG2)* were used as duplex targets for triplex formation by a series of oligonucleotides. Corresponding oligonucleotides pso-AG10, pso-AGT30, and pso-AGT43 were designed to bind in the anti-parallel motif to the 10, 30, and 43 bp sites in these genes. Pso-AGT20, designed to bind to bp 167-186 in *supFGla,* was also tested. Fixed concentrations of radioactively labelled duplex target DNA were incubated with increasing concentrations of the psoralen-linked oligomers to assay for triplex formation over a range of concentrations. UVA irradiation (1.8 J/cm2) was used to generate photoadducts and thereby covalently link the mutagenic oligonucleotides to their targets, ensuring that subsequent manipulation of the samples would not alter the apparent binding. The samples were then analyzed by denaturing gel electrophoresis and autoradiography.

Analyses of the binding of pso-AG10 and pso-AGT30 to their respective sites in the *supF* and *supFGla* genes demonstrated that photoactivation of the triplex molecules with 1.8 J/cm² of UVA leads to a high proportion of cross-links (the psoralen-mutagenic oligonucleotide bound covalently to both strands of the duplex target via a psoralen interstrand cross-link - the XL band) and to a small proportion of psoralen monoadducts (the psoralen-mutagenic oligonucleotide linked covalently linked to just one strand of the duplex via psoralen monoadduct formation - the MA band). The percentage of the sample constituting the sum of the XL and MA bands is proportionate to the extent of triple helix formation. When oligonucleotides that cannot form triplex at the target site are used in this assay, no XL or MA bands are visualized (not shown), and so adducts can be taken as indicative of triplex formation. As the concentration of the triplex-forming oligonucleotide is increased, the proportion of the target duplex bound as either MA or XL, as opposed to unbound, increases. As can be seen from the graphical analysis in Fig. 9, the concentration dependence of triplex formation by pso-AG10 and by pso-AGT30 are quite different. A useful measure of triple helix formation is the equilibrium dissociation constant, K_{d}, which can be estimated as the concentration of mutagenic oligonucleotide at which triplex formation is half-maximal. For pso-AG10 the K_{d} is 8 x 10⁻⁷ M, whereas for pso-AGT30, the K_{d} is 3 x 10⁻⁹ M, a 270-fold difference. The greater affinity for triplex-formation by pso-AGT30 is consistent with the results of other studies correlating oligonucleotide length and binding affinity, and it places the affinity of pso-AGT30 for binding to *supFGla* in the range of physiologic interactions. Similar analyses revealed a K_{d} of 1 x 10⁻⁹ M for pso-AGT43 binding to *supFG2* and 8 x 10-9 M for pso-AGT20 binding to *supFGla.*

**Targeted mutagenesis *in vivo*.** Using pSupFGla as a target vector, experiments were conducted to study intracellular mutation targeting by three psoralen-linked oligonucleotides, all of which were designed to form a triple helix with sequences in *supFGla* and to deliver the tethered psoralen to bp 166-167. However, the oligomers differed in length and binding affinity for triplex formation as shown in Table 3 below. In these experiments, the oligomers were added to the cells approximately one hour following electroporation with the vector DNA. The cells were then irradiated with UVA either two hours or eight hours after oligonucleotide addition. At both the two and eight hour time points, the extent of mutagenesis in the *supFGla* gene was seen to depend on the measured strength of triplex formation by the respective mutagenic oligonucleotide. At two hours, pso-AG10 produced little mutagenesis above background, whereas pso-AGT30 induced mutations in 1-2% of the vector molecules. At the eight hour point, some induced mutagenesis was seen with pso-AG10, while psoAGT30 produced mutations in 5% of the target genes. The level of mutagenesis produced by pso-AGT20 was intermediate between the two. These results help to define the requirements for efficient *in vivo* triplex formation and mutation targeting.

**TABLE 3.**

| Targeted mutagenesis of pSupFG1a within COS cells | | | | | |
|---|---|---|---|---|---|
| Oligo | K_{d} | Conc. µM | Time of UVA irradiation (hrs) | Mutation frequency | Mutants per total colonies |
| None | n.a. | n.a. | n.a. | 0.03 | 2/7,600 |
| | | | | | |
| pso-AG10 | 8 x 10⁻⁷ M | 0.9 | 2 | 0.05 | 4/8,674 |
| | | 0.2 | 8 | 0.06 | 2/3,148 |
| | | | | | |
| pso-AGT20 | 7 x 10⁻⁹ M | 0.45 | 2 | 0.33 | 6/18,00 |
| | | 2.0 | 8 | 1.2 | 13/1073 |
| | | | | | |
| pso-AGT30 | 3 x 10⁻⁹ M | 0.45 | 2 | 0.99 | 51/5,175 |
| | | 2.0 | 8 | 5.3 | 343/651 |

**Time course of UVA irradiation.** The kinetics of intracellular targeted mutagenesis mediated by the mutagenic oligonucleotide was investigated. This process depends on the entry of the oligonucleotides into the cells, migration into the nucleus, and specific binding to the triplex target site. In order to achieve targeted mutagenesis via site-specific generation of a psoralen photoadduct, these steps must occur by the time the UVA irradiation is given. To determine the optimal time for UVA irradiation, a time course experiment was carried out in which the time of UVA irradiation following pso-AGT30 addition to the cell culture medium was varied. The results are shown in Table 4. As the time allowed for intracellular triplex formation before UVA irradiation was increased, the yield of targeted mutations also increased, with a frequency as high as 5.3% for the 8 hour point. Given sufficient time, therefore, the psoralen-linked oligonucleotides can enter cells, form a site-specific triple helix, and mediate targeted mutagenesis of an SV40 vector at frequencies in the range of 5%.

At the later time points, it is theoretically possible that lesions are targeted to vectors that are not subsequently replicated or repaired before the molecules are transformed into bacteria for analysis. Hence, bacterial processing of persistent lesions produced in the COS cells may be occurring. Such vector molecules could escape the *Dpn* I restriction step designed to eliminate input vector molecules that had not replicated in the COS cells if they had already undergone replication prior to the triplex formation and UVA irradiation. However, to address this possibility, we allowed pso-AGT30 and pSupFGla to form triplex *in vitro,* irradiated the complex with 1.8 J/cm² of UVA irradiation, and used the sample to directly transform *E. coli* without passage through COS cells. A mutation frequency of only 0.03% (4/15,000) was seen. Therefore, the targeted mutagenesis observed in this experiment cannot be accounted for by the rescue of unprocessed vector molecules form the COS cells.

**TABLE 4.**

| Time dependence of targeted mutagenesis within COS cells | | | |
|---|---|---|---|
| Oligonucleotide^{*a*} | Time of UVA after oligo addition (hrs) | Mutation frequency % | Mutants/total |
| None | n.a. | 0.03 | 4/14,700 |
| pso-AGT30 | 1 | 1.1 | 101/9,400 |
| " | 2 | 2.1 | 178/8,663 |
| " | 4 | 3.6 | 474/13,339 |
| " | 8 | 5.3 | 343/6,515 |

| | | | |
|---|---|---|---|
| ^{*a*} The oligonucleotide concentration used was 2 µM for all time points. | | | |

**Time course of oligonucleotide treatment.** In the above experiments, the cells were exposed to the mutagenic oligonucleotide within one hour after introduction of the SV40 vector DNA by electroporation. Although transfected SV40 vectors become covered in chromatin upon introduction into monkey cells, this process could be incomplete at such an early time point. An experiment was performed in which the cells were transfected with pSupFGla, allowed to attach to dishes in growth medium, and incubated for 12 hours. The cells were then detached by trypsinization, washed three times in growth medium and incubated in the presence of 2 µM pso-AGT30 for two hours before irradiation with UVA. In this experiment, mutations were generated in the *supFGla* gene at a frequency of 1.5% (150/10,175), in the same range as the frequency at the early time point for the same concentration of oligonucleotide (2.1 %). Hence, even with more than enough time allowed for chromatin assembly on the SV40 vector DNA, the psoralen-conjugated oligonucleotide can still generate targeted mutations in the *supFGla* gene within the vector. This provides evidence that triplex formation can occur with chromatin.

The detection of targeted mutations in this experiment also confirms that the targeted mutagenesis that observed is mediated by intracellular triplex formation. After growth of the cells for 12 hours, trypsinization, dilution, and extensive washing, it would be unlikely that any vector DNA would persist in the extracellular medium at the time of oligonucleotide treatment.

**Concentration dependence.** The concentration dependence for targeted mutagenesis was also investigated. The results are shown in Table 5 below. Following electroporation of the COS cells with the SV40 vector DNA, the cells were incubated in the presence of pso-AGT30 at concentrations from 0.1 nM to 2 µM. UVA irradiation was given eight hours later. As can be seen, a low but detectable frequency of mutagenesis was observed with the extracellular oligonucleotide concentration in the nanomolar range. This is consistent with the K_{d} for triplex formation for pso-AGT30 and supFGla being 3 x 10⁻⁹ M. Although intracellular oligonucleotide concentrations were not directly measured in these experiments, other studies have reported that treatment of mammalian cells with oligonucleotides produces concentrations within cells that are in the same range as the given extracellular concentrations. Significant levels of targeted mutagenesis were seen at an extracellular mutagenic oligonucleotide concentration of 1 µM, consistent with the data presented in Tables 3 and 4.

**TABLE 5.**

| Concentration dependence of the targeted mutagenesis within COS cells | | | |
|---|---|---|---|
| Oligonucleotide | Concentration (nM) (hrs) | Mutation frequency^{a} % | Mutants/total |
| None | 0 | 0.06 | 2/3,400 |
| pso-AGT30 | 0.1 | 0.3 | 6/2,050 |
| " | 1 | 0.6 | 12/2,200 |
| " | 10 | 0.8 | 20/2,550 |
| " | 100 | 0.7 | 19/2,860 |
| " | 1000 | 2.3 | 198/8771 |
| " | 2000 | 5.3 | 343/6515 |

| | | | |
|---|---|---|---|
| ^{*a*} The values represent the frequency of mutations induced at the oligonucleotide concentrations listed with UVA irradiation given eight hours after oligonucleotide addition to the cells. | | | |

**Sequence analysis.** A series of 36 mutations induced in the *supF* gene by pso-AGT30 at the 2 hour and 8 hour time points were analyzed by DNA sequencing. The results are shown in Fig. 10. Of these, 28 (78%) were T:A to A:T transversions at bp 166 (the predicted psoralen intercalation site) while 6 (17%) were deletions of various sites spanning the triplex target site. The observed frequency of deletions may be an underestimate, since larger deletions involving sequences surrounding the *supFGla* gene would inactivate essential genes for vector replication. Therefore, molecules undergoing such deletions would not be detected in the assay. These results demonstrate the specificity of the intracellular mutation targeting and suggest that the psoralen adducts can generate either specific point mutations or deletions at target site.

**Specificity: the effect of partial homology for triplex formation.** The specificity of the mutagenic oligonucleotide-mediated targeted mutagenesis may be influenced by several factors, one of which is the existence of alternate sites in the DNA having partial homology for triplex formation by the psoralen-conjugated mutagenic oligonucleotide. This issue was examined by comparing targeted mutagenesis of the *supFGla* and *supFG2* genes by pso-AGT43. Pso-AGT43 is designed to form a triple helix at base pairs 167-209 of *supFG2.* In the anti-parallel motif, it matches exactly the 43 bp site in *supFG2* except for the two T interruptions at base pairs 180 and 183 (95% homology). In contrast, pso-AGT43 has only 65% homology for triplex formation with *supFGla.* The first 30 nucleotides in pso-AGT43 match exactly those in pso-AGT30, and so pso-AGT43 has 28 out of 43 nucleotide homology for triplex formation with *supFGla* (taking into account the unavoidable mismatches at 180 and 183). As with pso-AGT30, the tethered psoralen in pso-AGT43 is targeted to intercalate at base pairs 166-167. In conceptually similar experiments, a comparison of pso-AGT30 in targeting both the *supFGla* gene (to which it is designed to form triplex) and the original *supF* gene (to which it has only 10 out of 30 nucleotide homology for triplex formation) was made. In these experiments, the electroporated COS cells were incubated in 2 µM concentrations of the mutagenic oligonucleotide for two hours before UVA irradiation was given.

Pso-AGT43 effectively targeted mutations to *supFGla* as shown below in Table 6. However, pso-AGT43 failed to induce mutations in the *supFGla* gene above the background frequency, suggesting that the partial homology for triplex formation with *supFGla* is insufficient to mediate significant *in vivo* interactions. In the same way, pso-AGT30 can target mutations to *supFGla,* but it is not effective in inducing mutations in the unmodified *supF.* These results demonstrate the specificity of the intracellular targeting.

The frequency of targeted mutations induced by pso-AGT43 in *supFG2* was not higher than that seen with pso-AGT30 and *supFGla,* in contrast to the increase observed in going from pso-AG10 to pso-AGT20 to pso-AGT-30 (Table 3). It is possible that the 43 nucleotide long G-rich oligonucleotide (pso-AGT43) may be subject to K⁺-driven self-association to form G-quartets, limiting its effectiveness within the cells.

**TABLE 6.**

| Target site specificity. | | | | |
|---|---|---|---|---|
| Oligonucleotide | Target gene | Homology for triplex formation^{a} | Mutation frequency^{b} % | Mutants/total |
| pso-ATG43 | *supFGla* | 30/43 | 0.07 | 7/6887 |
| pso-AGT43 | *supFG2* | 41/43 | 1.4 | 223/16,425 |
| pso-AGT30 | *supFGla* | 28/30 | 2.1 | 178/8663 |
| pso-AGT3O | *supF* | 17/30 | ≤0.02 | 0/5,300 |
| pso-TCGA30 | *supFGla* | 12/30 | 0.03 | 3/8,740 |

| | | | | |
|---|---|---|---|---|
| ^{a} The values represent the fraction of base matches in the anti-parallel triple helix motif (G for G:C bp and either A or T for A:T bp) between the listed oligonucleotide and the designated target gene. | | | | |
| ^{b} The values represent the frequency of induced mutations observed using oligonucleotide concentrations of 2 µM and with UVA irradiation given two hours after oligonucleotide addition to the cells. | | | | |

The foregoing results demonstrate use of a triple helix forming oligonucleotide to generate targeted mutations within mammalian cells. Linkage of the oligonucleotide to a mutagen, psoralen, confers sequence specificity on the action of the psoralen, which is delivered to the selected site via intracellular triple helix formation. An oligomer with a K_{d} in the range of 10⁻⁹ M or better is preferred for significant intracellular interactions. Other factors that might affect triplex formation under physiologic conditions, such as oligonucleotide base composition and backbone structure, could be tested using this type of assay. Because selected sequences within and adjacent to the *supF* gene can be manipulated without disrupting suppressor activity, additional target sites designed to specifically examine other aspects of intracellular triplex formation could be constructed.

Sequence analysis of the mutations targeted by pso-AGT30 revealed a high specificity for T:A to A:T transversions at the predicted psoralen intercalation site (bp 166). The results of the time course experiment indicate that the processes of oligonucleotide entry into cells and of intracellular formation of triple helices occur over several hours. The increased frequency of mutations seen at the later time points may, in part, reflect the time needed for these processes as well as the interplay of repair and replication in forming the targeted DNA lesions into mutations.

Modifications and variations of the present invention, mutagenic triplex-forming oligonucleotides, as well as methods of use thereof, will be obvious to those skilled in the art from the foregoing detailed description. Such modifications and variations are intended to come within the scope of the appended claims.

## Claims

1. A composition comprising a mutagenic oligonucleotide for site-directed mutagenesis of a double-stranded nucleic acid molecule comprising a mutagen incorporated into a single-stranded (oligonucleotide between 7 and 40 nucleotides in length) having a sequence that forms a triple-stranded nucleic acid molecule with a target region of the double-stranded nucleic acid molecule, and a pharmaceutically acceptable carrier for administration to cells.

2. The composition of claim 1 wherein the mutagen is selected from the group consisting of psoralen, acridine orange, an alkylating agent, a cis-platinum analog, a hematoporphyrin, a hematoporphyrin derivative, mitomycin C, a radionuclide, and a molecule that interacts with radiation to become mutagenic.

3. The composition of claim 1 wherein the mutagen causes a mutation in the double-stranded nucleic acid molecule in the presence of light.

4. The composition of claim 3 wherein the mutagenic chemical is 4'hydroxymethyl-4,5',8-trimethylpsoralen.

5. The composition of claim 1 wherein the oligonucleotide has a length of between 20 and 30 nucleotide bases.

6. An ex vivo method for site-directed mutagenesis of a nucleic acid molecule in situ in a cell comprising the steps of:
a) selecting a target region of the nucleic and molecule
b) introducing a mutagenic oligonucleotide into the cell
c) hybridizing a mutagenic oligonucleotide to a target region of a double-stranded nucleic acid molecule, wherein the mutagenic oligonucleotide comprises a mutagen incorporated into a single-stranded (oligonucleotide between 7 and 40 nucleotides in length) that forms a triple-stranded nucleic acid molecule with the target region; and
d) mutating the double-stranded nucleic acid molecule.

7. The method of claim 6 comprising the additional step of activating the mutagen prior to the mutation step.

8. The method of claim 6 wherein the mutagen is selected from the group consisting of psoralen and acridine orange and is activated by light.

9. The method of claim 6 wherein the mutagen is selected from the group consisting of acridine orange, an alkylating agent, a cis-platinum analog, a hematoporphyrin, a hematoporphyrin derivative, mitomycin C, a radionuclide, and a molecule that interacts with radiation to become mutagenic.

10. The method of claim 6 wherein the mutation alters the activity of the double-stranded nucleic acid molecule.

11. The method of claim 6 wherein the double-stranded nucleic acid molecule is a gene, for example an oncogene, or a defective gene, for example a defective human β-hemoglobin gene.

12. The method of claim 6 wherein the double-stranded nucleic acid molecule is all or a portion of a viral genome.

13. A method of producing a composition of claim 1 the method comprising the steps of:
a) synthesizing an oligonucleotide substantially complementary based on the third strand binding code to a target region of a double-stranded nucleic acid molecule; and
b) incorporating a mutagen in the oligonucleotide, and c) admixing the mutagenic oligonucleotide with a pharmaceutically acceptable carrier for administration to cells.

14. The method of claim 13 wherein the mutagen is covalently linked to the oligonucleotide.

15. The method of claim 13 wherein the mutagen is incorporated into the oligonucleotide during synthesis of the oligonucleotide.

16. The method of claim 13 wherein the mutagen is bound to the oligonucleotide by photoactivation.

17. The method of claim 16 wherein the mutagen is selected from the group consisting of psoralen, acridine orange, an alkylating agent, a cis-platinum analog, a hematoporphyrin, a hematoporphyrin derivative, mitomycin C, a radionuclide, and a molecule that interacts with radiation to become mutagenic.

18. A mutagenic oligonucleotide for site-directed mutagenesis of a double-stranded nucleic acid molecule comprising a mutagen incorporated into a single-stranded (oligonucleotide between 7 and 40 nucleotides in length) having a sequence that forms a triple-stranded nucleic acid molecule with a target region of the double-stranded nucleic acid molecule for use in medicine.

19. Use of a mutagenic oligonucleotide comprising a mutagen incorporated into a single-stranded (oligonucleotide between 7 and 40 nucleotides in length) in the manufacture of a medicament for treating a cell or a patient with cell or which patient will benefit from site-directed mutagenesis of a double-stranded nucleic acid molecule, wherein the target region of said nucleic acid molecule is selected prior to treatment and the said oligonucleotide has a sequence that forms a triple-stranded nucleic acid molecule with a target region of the double-stranded nucleic acid molecule.

20. Use of a mutagenic oligonucleotide as defined in claim 1 for ex vivo site-directed mutagenesis of a nucleic acid molecule in situ in a cell.

## Patentansprüche

1. Zusammensetzung, die ein mutagenes Oligonukleotid für eine stellengerichtete Mutagenese eines doppelsträngigen Nukleinaäuremoleküls, welches Oligonukleotid ein in ein einzelsträngiges Oligonukleotid mit einer Länge von 7 bis 40 Nukleotiden, das eine Sequenz aufweist, die mit einem Targetbereich eines doppelsträngigen Nukleinsäuremoleküls ein dreisträngiges Nukleinsäuremolekül bildet, eingebautes Mutagen umfaßt, und einen pharmazeutisch akzeptablen Träger zur Verabreichung an Zellen enthält.

2. Zusammensetzung nach Anspruch 1, wobei das Mutagen unter Psoralen, Acridinorange, Alkylierungsmitteln, Cis-Platin-Analogen, Hämatoporphyrin, Hämatoporphyrinderivaten, Mitomycin C, Radionukliden und Molekülen, die mit Strahlung in Wechselwirkung treten, so daß sie mutagen werden, ausgewählt ist.

3. Zusammensetzung nach Anspruch 1, wobei das Mutagen eine Mutation in dem doppelsträngigen Nukleinsäuremolekül in Gegenwart von Licht hervorruft.

4. Zusammensetzung nach Anspruch 3, wobei die mutagene Chemikalie 4'-Hydroxymethyl-4,5',8-trimethylpsoralen ist.

5. Zusammensetzung nach Anspruch 1, wobei das Oligonukleotid eine Länge zwischen 20 und 30 Nukleotidbasen aufweist.

6. Ex-vivo-Verfahren zur stellengerichteten Mutagenese eines Nukleineäuremoleküls in situ in einer Zelle durch
a) Auswählen eines Targetbereichs des Nukleinsäuremoleküls;
b) Einführen eines mutagenen Oligonukleotids in die Zelle;
c) Hybridisieren eines mutagenen Oligonukleotids an einen Targetbereich eines doppelsträngigen Nukleinsäuremoleküls, wobei das mutagene Oligonukleotid ein in ein einzelsträngiges Oligonukleotid mit einer Länge von 7 bis 40 Nukleotiden, das mit dem Targetbereich ein dreisträngiges Nukleinsäuremolekül bildet, eingebautes Mutagen umfaßt; und
d) Mutieren des doppelsträngigen Nukleinsäuremoleküls.

7. Verfahren nach Anspruch 6, das die zusätzliche Stufe des Aktivierens des Mutagens vor der Mutationsstufe umfaßt.

8. Verfahren nach Anspruch 6, wobei das Mutagen unter Psoralen und Akridinorange ausgewählt wird und durch Licht aktiviert wird.

9. Verfahren nach Anspruch 6, wobei das Mutagen unter Akridinorange, Alkylierungsmitteln, Cis-Platin-Analogen, Hämatoporphyrin, Hämatoporphyrinderivaten, Mitomycin C, Radionukliden und Molekülen, die mit Strahlung in Wechselwirkung treten, so daß sie mutagen werden, ausgewählt wird.

10. Verfahren nach Anspruch 6, wobei die Mutation die Aktivität des doppelsträngigen Nukleinsäuremoleküls verändert.

11. Verfahren nach Anspruch 6, wobei das doppelsträngige Nukleinsäuremolekül ein Gen, beispielsweise ein Onkogen, oder ein Defektgen, beispielsweise ein defektes Humanbetahämoglobingen, ist.

12. Verfahren nach Anspruch 6, wobei das doppelsträngige Nuleinsäuremolekül die Gesamtheit oder ein Teil eines viralen Genoms ist.

13. Verfahren zur Herstellung einer Zusammensetzung nach Anspruch 1, durch
a) Synthetisieren eines basierend auf dem Bindungscode des dritten Strangs im wesentlichen zu einem Targetbereich eines doppelsträngigen Nukleinsäuremoleküls komplementären Oligonukleotids;
b) Einbauen eines Mutagens in das Oligonukleotid; und
c) Vermischen des mutagenen Oligonukleotids mit einem pharmazeutisch akzeptablen Träger zur Verabreichung an Zellen.

14. Verfahren nach Anspruch 13, wobei das Mutagen kovalent an das Oligonukleotid gebunden ist.

15. Verfahren nach Anspruch 13, wobei das Mutagen in das Oligonukleotid während der Synthese des Oligonukleotids eingebaut wird.

16. Verfahren nach Anspruch 13, wobei das Mutagen durch Fotoaktivierung an das Oligonukleotid gebunden wird.

17. Verfahren nach Anspruch 16, wobei das Mutagen unter Psoralen, Akridinorange, Alkylierungsmitteln, Cis-Platin-Analogen, Hämatoporphyrin, Hämatoporphyrinderivaten, Mitomycin C, Radionukliden und Molekülen, die mit Strahlung in Wechselwirkung treten, so daß sie mutagen werden, ausgewählt wird.

18. Mutagenes Oligonukleotid zur stellengerichteten Mutagenese eines doppelstrangigen Nukleinsäuremoleküls, das ein in ein einzelsträngiges Oligonukleotid mit einer Länge zwischen 7 und 40 Nukleotiden, das eine Sequenz aufweist, die mit einem Targetbereich des doppelsträngigen Nukleinsäuremoleküls ein dreisträngiges Nukleinsäuremolekül bildet, eingebautes Mutagen umfaßt, zur Verwendung in der Medizin.

19. Verwendung eines mutagenen Oligonukleotids, das ein in ein einzelsträngige Oligonukleotid mit einer Länge zwischen 7 und 40 Nukleotiden eingebautes Mutagen enthält, bei der Herstellung eines Medikaments zur Behandlung einer Zelle oder eines Patienten, welcher zelle oder welchem Patienten eine derartige stellengerichtete Mutagcnese eines doppelsträngigen Nukleinsäuremoleküls nützt, wobei der Targetbereich des Nukleinsäuremoleküls vor der Behandlung ausgewählt wird und wobei das Oligonukleotid eine Sequenz aufweist, die mit einem Targetbereich des doppelsträngigen Nukleinsäuremoleküls ein dreisträngiges Nukleinsäuremolekül bildet.

20. Verwendung eines mutagenen Oligonukleotids gemäß der Definition in Anspruch 1 zur stellengerichteten ex-vivo-Mutagenese eines Nukleinsäuremoleküls in situ in einer Zelle.

## Revendications

1. Composition contenant un oligonucléotide mutagène destiné à la mutagenèse dirigée vers un site d'une molécule d'acide nucléique à deux brins comprenant un mutagène incorporé dans un oligonucléotide à un brin ayant une longueur de 7 à 40 nucléotides et possédant une séquence qui forme une molécule d'acide nucléique à trois brins avec une région cible de la molécule d'acide nucléique à deux brins, ainsi qu'un substrat pharmaceutiquement acceptable pour l'administration à des cellules.

2. Composition selon la revendication 1, dans laquelle le mutagène est choisi dans le groupe comprenant le psoralène, l'orange d'acridine, un agent d'alkylation, un analogue du cisplatine, une hématoporphyrine, un dérivé de l'hématoporphyrine, la mitomycine C, un radionucléide, et une molécule réagissant avec un rayonnement pour devenir mutagène.

3. Composition selon la revendication 1, dans laquelle le mutagène provoque une mutation de la molécule d'acide nucléique à deux brins en présence de lumière.

4. Composition selon la revendication 3, dans laquelle la substance chimique mutagène est le 4'-hydroxyméthyl-4,5',8-triméthylpsoralène.

5. Composition selon la revendication 1, dans laquelle l'oligonucléotide a une longueur comprise entre 20 et 30 bases de nucléotides.

6. Procédé ex vivo de mutagenèse dirigée vers un site d'une molécule d'acide nucléique in situ dans une cellule, comprenant les étapes consistant à :
a) choisir une région cible de la molécule d'acide nucléique ;
b) introduire dans la cellule un oligonucléotide mutagène ;
c) hybrider un oligonucléotide mutagène à une région cible d'une molécule d'acide nucléique à deux brins, l'oligonucléotide mutagène comprenant un mutagène incorporé dans un oligonucléotide à un brin ayant une longueur de 7 à 40 nucléotides qui forme une molécule d'acide nucléique à trois brins avec la région cible ; et
d) effectuer la mutation de la molécule d'acide nucléique à deux brins.

7. Procédé selon la revendication 6, comprenant une étape supplémentaire d'activation du mutagène avant l'étape de mutation.

8. Procédé selon la revendication 6, dans lequel le mutagène est choisi dans le groupe comprenant le psoralène et l'orange d'acridine et est activé par la lumière.

9. Procédé selon la revendication 6, dans lequel le mutagène est choisi dans le groupe comprenant l'orange d'acridine, un agent d'alkylation, un analogue du cisplatine, une hématoporphyrine, un dérivé de l'hématoporphyrine, la mitomycine C, un radionucléide, et une molécule réagissant avec un rayonnement pour devenir mutagène.

10. Procédé selon la revendication 6, dans lequel la mutation modifie l'activité de la molécule d'acide nucléique à deux brins.

11. Procédé selon la revendication 6, dans lequel la molécule d'acide nucléique à deux brins est un gène, par exemple un oncogène, ou bien un gène défectueux, par exemple un gène défectueux de la β-hémoglobine humaine.

12. Procédé selon la revendication 6, dans lequel la molécule d'acide nucléique à deux brins constitue la totalité ou une partie d'un génome viral.

13. Procédé de production d'une composition selon la revendication 1, ledit procédé comprenant les étapes consistant à :
a) synthétiser un oligonucléotide substantiellement complémentaire en se basant sur le code de liaison du troisième brin à une région cible d'une molécule d'acide nucléique à deux brins ;
b) incorporer un mutagène dans l'oligonucléotide ; et
c) mélanger l'oligonucléotide mutagène avec un substrat pharmaceutiquement acceptable pour l'administration à des cellules.

14. Procédé selon la revendication 13, dans lequel le mutagène est lié de manière covalente à l'oligonucléotide.

15. Procédé selon la revendication 13, dans lequel le mutagène est incorporé dans l'oligonucléotide lors de la synthèse dudit oligonucléotide.

16. Procédé selon la revendication 13, dans lequel le mutagène est lié à l'oligonucléotide par photoactivation.

17. Procédé selon la revendication 16, dans lequel le mutagène est choisi dans le groupe comprenant le psoralène, l'orange d'acridine, un agent d'alkylation, un analogue du cisplatine, une hématoporphyrine, un dérivé de l'hématoporphyrine, la mitomycine C, un radionucléide, et une molécule réagissant avec un rayonnement pour devenir mutagène.

18. Oligonucléotide mutagène destiné à la mutagenèse dirigée sur un site d'une molécule d'acide nucléique à deux brins comprenant un mutagène incorporé dans un oligonucléotide à un brin ayant une longueur de 7 à 40 nucléotides et possédant une séquence qui forme une molécule d'acide nucléique à trois brins avec une région cible de la molécule d'acide nucléique à deux brins pour une utilisation en médecine.

19. Utilisation d'un oligonucléotide mutagène comprenant un mutagène incorporé dans un oligonucléotide à un brin ayant une longueur de 7 à 40 nucléotides pour la fabrication d'un médicament destiné au traitement d'une cellule ou d'un patient, ladite cellule ou ledit patient bénéficiant de la mutagenèse dirigée sur un site d'une molécule d'acide nucléique à deux brins, dans laquelle la région cible de ladite molécule d'acide nucléique est choisie avant le traitement et ledit oligonucléotide possède une séquence qui forme une molécule d'acide nucléique à trois brins avec une région cible de la molécule d'acide nucléique à deux brins.

20. Utilisation d'un oligonucléotide mutagène tel que défini dans la revendication 1 pour la mutagenèse dirigée sur un site ex vivo d'une molécule d'acide nucléique in situ dans une cellule.
